# EUROPEAN PATENT SPECIFICATION

(11) **EP 3 755 217 B1**
(45) Date of publication and mention of the grant of the patent: **28.01.2026**
(21) Application number: 19756660.7
(22) Date of filing: 20.02.2019
(51) Int. Cl.: A61B 5/0245, A61B 5/273

(54) **CONNECTION SYSTEMS FOR ESTABLISHING ELECTRICAL CONNECTIONS ACROSS A STERILE FIELD**
VERBINDUNGSSYSTEME ZUR HERSTELLUNG ELEKTRISCHER VERBINDUNGEN ÜBER EIN STERILES FELD
SYSTÈMES DE CONNEXION POUR ÉTABLIR DES CONNEXIONS ÉLECTRIQUES DANS UN CHAMP STÉRILE

(30) Priority: 20.02.2018 US 201862632608 P; 29.01.2019 US 201916261368
(43) Date of publication of application: 30.12.2020
(73) Proprietor: Bard Access Systems, Inc., Salt Lake City, UT 84116 (US)
(72) Inventor: BARRON, William, R., Riverton, UT 84065 (US); SNYDER, Scott, Walter, West Valley City, UT 84128 (US); WILEY, Martha, R., Salt Lake City, UT 84108 (US); MCLAUGHLIN, William, Robert, Bountiful, UT 84010 (US); ZENTGRAF, Bradley, W., West Jordan, UT 84084 (US); MISENER, Anthony, Kent, Bountiful, UT 84010 (US)
(74) Representative: Hoffmann Eitle
(86) International application number: PCT/US2019/018851
(87) International publication number: WO 2019/165011

(56) References cited:
- EP-A1- 0 484 107
- WO-A1-2017/021834
- WO-A1-98/22180
- DE-U1- 20 020 036
- US-A- 3 329 928
- US-A- 4 369 794
- US-A- 4 700 997
- US-A- 5 325 868
- US-A- 5 560 358
- US-A1- 2002 197 905
- US-A1- 2004 230 268
- US-A1- 2008 046 062
- US-A1- 2009 234 328
- US-A1- 2011 250 775
- US-A1- 2011 294 358
- US-A1- 2011 294 360
- US-A1- 2013 023 159
- US-A1- 2013 211 225
- US-A1- 2013 289 417
- US-A1- 2014 364 713
- US-A1- 2015 082 623
- US-B1- 8 147 275

## Description

### PRIORITY

This application is a continuation-in-part of U.S. Patent Application No. 16/261,368, filed January 29, 2019, which claims the benefit of priority to U.S. Provisional Patent Application No. 62/623,394, filed January 29, 2018. This application also claims the benefit of priority to U.S. Provisional Patent Application No. 62/632,608, filed February 20, 2018.

### BACKGROUND

In a typical surgical procedure, a sterile drape is placed over a patient to establish a sterile field, within which the surgical procedure is performed. For example, in a typical catheter-placement procedure, a sterile drape is placed over a patient to establish a sterile field for placement of the catheter. However, there is often a need to breach the sterile barrier in order to make electrical connections between components of various systems across the sterile field without compromising the sterility of the sterile field. Disclosed herein are connection systems for establishing electrical connections across a sterile field and methods thereof that address at least the foregoing need.

### SUMMARY

According to the present invention, there is provided a connection system as defined in claim 1. The dependent claims define preferred embodiments. According to the invention, the probe-connecting means is an actuatable device including an actuator configured to actuate a connecting mechanism of the actuatable device for establishing the second electrical connection between the electrical contact(s) and the wire probe within the housing.

Disclosed herein is a connection system for establishing electrical connections across a sterile field, the connection system including, in some embodiments, a sensor connector, a probe connector, and a tether having a first end connected to the sensor connector and a second end connected to the probe connector. The sensor connector includes a piercing element. The piercing element is configured to pierce a sterile side of a drape and establish a first electrical connection with a receptacle of a sensor when the piercing element is inserted into the receptacle. The probe connector includes a probe-connecting means configured for a user to establish a second electrical connection between an electrical contact of the probe connector and a wire probe. The tether is configured to convey electrical signals between the sensor connector and the probe connector.

In some embodiments, the probe-connecting means is a non-directional clamp configured to clamp the wire probe in any user-chosen orientation of a nearly unlimited number of orientations of the wire probe to the non-directional clamp.

In some embodiments, the probe-connecting means is a semi-directional clamp configured to clamp the wire probe in any user-chosen orientation of a limited number of orientations of the wire probe to the semi-directional clamp.

In some embodiments, the probe-connecting means 1s a directional clamp configured to clamp the wire probe in a particular orientation of the wire probe to the non-directional clamp.

In some embodiments, the probe-connecting means is a threadable substrate including structures configured for threading the wire probe through the threadable substrate.

In some embodiments, the structures of the threadable substrate are one or more holes, poles, or a combination thereof.

In some embodiments, the probe-connecting means includes a threaded substrate configured for screwing the wire probe into the substrate.

In some embodiments, the probe-connecting means is a self-actuatable device configured to actuate a retaining mechanism of the self-actuatable device for retaining the second electrical connection with the wire probe.

In some embodiments, the probe-connecting means is formed of a metal or a polymer. The probe-connecting means formed of the polymer has the electrical contact disposed therein for establishing the second electrical connection with the wire probe.

In the invention, the wire probe is a guidewire.

In some embodiments, the probe connector includes a slideable insert disposed in the housing. The probe connector also includes an open-clamp position of the slideable insert and a closed-clamp position of the slideable insert.

In some embodiments, the slideable insert is configured to extend past an end of the housing in the open-clamp position of the slideable insert and be substantially concealed within the housing in the closed-clamp position of the slideable insert.

In some embodiments, the slideable insert is configured to extend from a first side of the housing in the open-clamp position of the slideable insert and extend from a second side of the housing opposite the first side in the closed-clamp position of the slideable insert.

In some embodiments, the probe connector includes the electrical contact within the housing. The probe connector also includes an open position of the clamp with a jaw of the clamp substantially extending from a side of the housing and a closed position of the clamp with the jaw of the clamp substantially extending into the housing adjacent the electrical contact or against the electrical contact.

In some embodiments, the probe connector incorporates the clamp into the housing. The probe connector also includes an open-clamp position of the housing and a closed-clamp position of the housing.

In some embodiments, the actuator is a knob or handle extending from a side of the housing. The knob or handle is configured to rotate until the second electrical connection is established between the electrical contact within the housing and the wire probe.

In some embodiments, the actuator is a push button extending from a side of the housing. The push button is configured to establish the second electrical connection between the electrical contact within the housing and the wire probe when the push button is pushed.

In some embodiments, the actuator is a slide button extending from a side of the housing. The slide button is configured to establish the second electrical connection between the electrical contact within the housing and the wire probe when the slide button is slid from a first side of a channel in the side of the housing to a second side of the channel opposite the first side.

In some embodiments, the actuator is a twistable end portion of the housing. The twistable end portion is configured to twist until the second electrical connection is established between the electrical contact within the housing and the wire probe.

These and other features of the concepts provided herein will become more apparent to those of skill in the art in view of the accompanying drawings and following description, which disclose particular embodiments of such concepts in greater detail.

### DRAWINGS

FIG. 1 illustrates a block diagram of a catheter-placement system for placing a catheter in a patient's body in accordance with some embodiments.
FIG. 2 illustrates the catheter-placement system and the patient in accordance with some embodiments.
FIG. 3A illustrates a connection system of a first type in accordance with some embodiments.
FIG. 3B illustrates a connection system of a second type in accordance with some embodiments.
FIG. 4 illustrates a first connection system including a first probe connector in accordance with some embodiments.
FIG. 5 illustrates a second connection system including a second probe connector in accordance with some embodiments.
FIG. 6 illustrates a third connection system including a third probe connector in accordance with some embodiments.
FIG. 7 illustrates a fourth connection system including a fourth probe connector in accordance with some embodiments.
FIG. 8 illustrates a fifth connection system including a fifth probe connector in accordance with some embodiments.
FIG. 9A illustrates a sixth connection system including a sixth probe connector in accordance with some embodiments.
FIG. 9B illustrates a longitudinal cross section of the sixth probe connector in accordance with some embodiments.
FIG. 10 illustrates a seventh connection system including a seventh probe connector in accordance with some embodiments.
FIG. 11A illustrates an eighth connection system including an eighth probe connector in accordance with some embodiments.
FIG. 11B illustrates a ninth connection system including a ninth probe connector in accordance with some embodiments.
FIG. 12A illustrates a tenth connection system including a tenth probe connector in accordance with some embodiments.
FIG. 12B illustrates a transverse cross section of the tenth probe connector in accordance with some embodiments.
FIG. 13A illustrates an eleventh connection system including an eleventh probe connector in accordance with some embodiments.
FIG. 13B illustrates a transverse cross section of the eleventh probe connector in accordance with some embodiments.
FIG. 14A illustrates a twelfth connection system including a twelfth probe connector in accordance with some embodiments.
FIG. 14B illustrates a longitudinal cross section of the twelfth probe connector in accordance with some embodiments.
FIG. 15A illustrates a thirteenth connection system including a thirteenth probe connector in accordance with some embodiments.
FIG. 15B illustrates a transverse cross section of the thirteenth probe connector in accordance with some embodiments.
FIG. 15C illustrates a longitudinal cross section of the thirteenth probe connector in accordance with some embodiments.
FIG. 16A illustrates a fourteenth connection system including a fourteenth probe connector in accordance with some embodiments.
FIG. 16B illustrates the fourteenth connection system with a wire probe in accordance with some embodiments.
FIG. 17A illustrates a fifteenth connection system including a fifteenth probe connector in accordance with some embodiments.
FIG. 17B illustrates a transverse cross section of the fifteenth probe connector in accordance with some embodiments.
FIG. 18A illustrates a sixteenth connection system including a sixteenth probe connector in accordance with some embodiments.
FIG. 18B illustrates a transverse cross section of the sixteenth probe connector in accordance with some embodiments.
FIG. 19A illustrates a first view of a seventeenth connection system including a seventeenth probe connector in accordance with some embodiments.
FIG. 19B illustrates a second view of the seventeenth connection system including the seventeenth probe connector in accordance with some embodiments.
FIG. 19C illustrates a connecting mechanism of the seventeenth probe connector in accordance with some embodiments.
FIG. 19D illustrates an alternative connecting mechanism of the seventeenth probe connector in accordance with some embodiments.
FIG. 20A illustrates an eighteenth connection system including an eighteenth probe connector in accordance with some embodiments.
FIG. 20B illustrates a transverse cross section of the eighteenth probe connector in accordance with some embodiments.
FIG. 21A illustrates a nineteenth connection system including a nineteenth probe connector in accordance with some embodiments.
FIG. 21B illustrates a longitudinal cross section of the nineteenth probe connector in accordance with some embodiments.
FIG. 22A illustrates a twentieth connection system including a twentieth probe connector in accordance with some embodiments.
FIG. 22B illustrates a longitudinal cross section of the twentieth probe connector in accordance with some embodiments.
FIG. 23 illustrates a twenty-first connection system including a twenty-first probe connector in accordance with some embodiments.
FIG. 24A illustrates a twenty-second connection system including a twenty-second probe connector in accordance with some embodiments.
FIG. 24B illustrates a transverse cross section of the twenty-second probe connector in accordance with some embodiments.
FIG. 25 illustrates a twenty-third connection system including a twenty-third probe connector in accordance with some embodiments.
FIG. 26A illustrates a twenty-fourth connection system including a twenty-fourth probe connector in accordance with some embodiments.
FIG. 26B illustrates disengaging a wire probe from the twenty-fourth probe connector in accordance with some embodiments.
FIG. 27A illustrates a twenty-fifth connection system including a twenty-fifth probe connector in accordance with some embodiments.
FIG. 27B illustrates disengaging a wire probe from the twenty-fifth probe connector in accordance with some embodiments.
FIG. 28A illustrates a twenty-sixth connection system including a twenty-sixth probe connector in accordance with some embodiments.
FIG. 28B illustrates a longitudinal cross section of the twenty-sixth probe connector in accordance with some embodiments.
FIG. 29A illustrates a twenty-seventh connection system including a twenty-seventh probe connector in accordance with some embodiments.
FIG. 29B illustrates a longitudinal cross section of the twenty-seventh probe connector in accordance with some embodiments.
FIG. 30A illustrates a twenty-eighth connection system including a twenty-eighth probe connector in an inactive state in accordance with some embodiments.
FIG. 30B illustrates the twenty-eighth connection system including the twenty-eighth probe connector in an active state in accordance with some embodiments.
FIG. 31 illustrates a wire-probe dispenser in accordance with some embodiments.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

Unless defined otherwise, all technical and scientific terms used herein have the same meaning as commonly understood by those of ordinary skill in the art.

In a typical surgical procedure, a sterile drape is placed over a patient to establish a sterile field, within which the surgical procedure is performed. For example, in a typical catheter-placement procedure, a sterile drape is placed over a patient to establish a sterile field for placement of the catheter. However, there is often a need to breach the sterile barrier in order to make electrical connections between components of various systems across the sterile field without compromising the sterility of the sterile field. Disclosed herein are connection systems for establishing electrical connections across a sterile field and methods thereof that address at least the foregoing need.

For example, a connection system for establishing electrical connections across a sterile field can include a sensor connector, a probe connector, and a tether having a first end connected to the sensor connector and a second end connected to the probe connector. The sensor connector can include a piercing element. The piercing element can be configured to pierce a sterile side of a drape and establish a first electrical connection with a receptacle of a sensor when the piercing element is inserted into the receptacle. The probe connector can include a probe-connecting means configured for a user to establish a second electrical connection between an electrical contact of the probe connector and a wire probe. The tether is configured to convey electrical signals between the sensor connector and the probe connector.

An example catheter-placement system incorporating the connection system will be at least initially described to provide a context for the connection system. It should be understood the connection system is not limited to the example catheter-placement system. Indeed, the connection system can be incorporated into any system of various systems having the need to breach a sterile barrier in order to make electrical connections between components of the system across a sterile field without compromising the sterility of the sterile field.

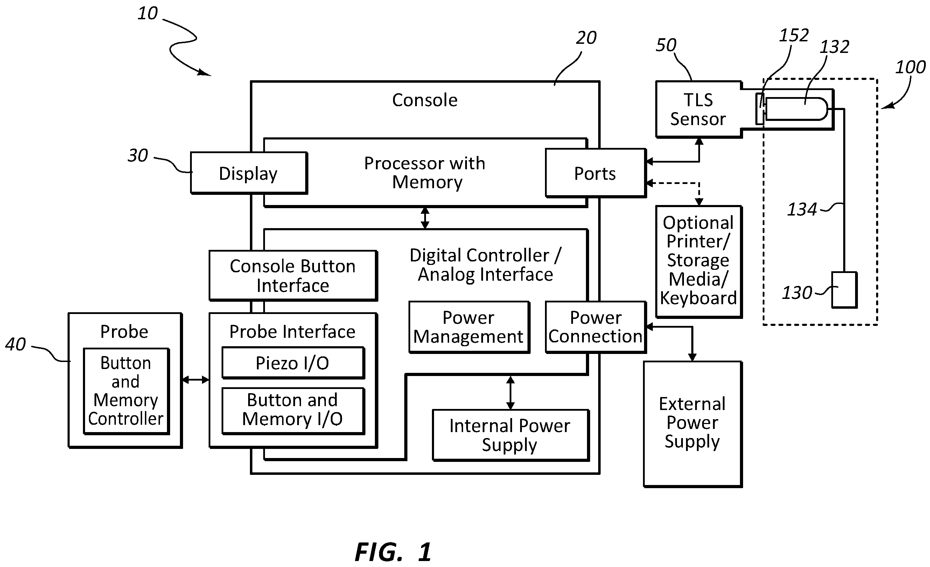

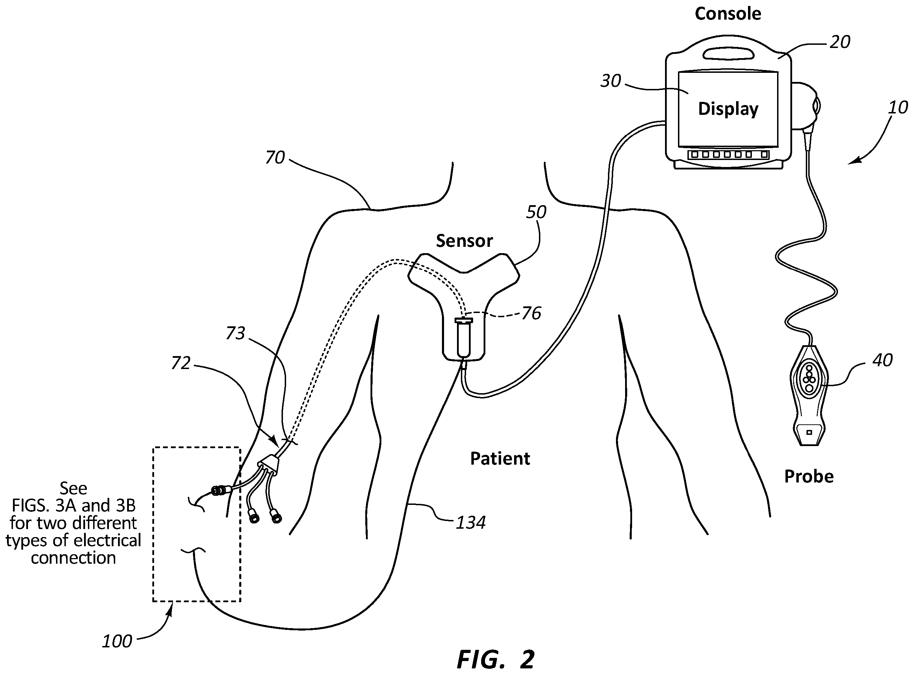

FIG. 1 illustrates a block diagram of a catheter-placement system 10 for placing a catheter 72 in a body of patient 70 in accordance with some embodiments. FIG. 2 illustrates the catheter-placement system 10 and the patient 70 in accordance with some embodiments. The catheter-placement system 10 is configured for assisting a clinician in placing the catheter 72 in a vasculature of the patient 70 through a percutaneous insertion site 73. As shown, the catheter-placement system 10 includes a console 20 including a display 30, an ultrasound probe 40, and a tip-location sensor 50 having a connector 152, wherein the tip-location sensor 50 is configured for placement on the patient's chest or some other portion of the patient's body outside a sterile field such as under a sterile drape. The catheter-placement system 10 also includes a connection system 100 for establishing electrical connections across the sterile field. The connection system 100 includes a sensor connector 132, a probe connector 130, and a tether 134 having an end connected to the sensor connector 132 and another end connected to the probe connector 130.

FIG. 2 also illustrates a wire probe such as a guidewire *GW* for use use with the catheter-placement system 10 in accordance with some embodiments. The guidewire is employed with the catheter 72 during insertion of the catheter 72 into the vasculature of the patient 70. The guidewire is configured to be removably disposed in a lumen of the catheter 72 during a catheter-placement procedure, thereby enabling a distal tip 76 of the catheter 72 to be tracked to a desired location within the patient's vasculature by the catheter-placement system 10 using one or more of modalities for guiding the catheter 72. The one or more modalities include, but are not limited to ultrasound-based imaging of subcutaneous tissue of the patient in preparation for insertion of the catheter 72; magnet-based tracking for determining orientation, advancement direction, and general internal location of the distal tip 76 of the catheter 72; or ECG-based confirmation for confirming the distal tip 76 of the catheter 72 is positioned at a desired location.

Additional details for the catheter-placement system 10 shown in FIGS. 1 and 2 can be found in U.S. Patent No. 9,649,048. Such additional details include those for the sensor connector 132, which includes a piercing element configured to pierce a sterile side of a drape and establish an electrical connection with a receptacle of a sensor when the piercing element is inserted into the receptacle. Such a receptacle includes a receptacle of the connector 152 of the tip-location sensor 50. Additional details for the sensor connector 132 can also be found in U.S. Patent Application No. 16/261,368, filed January 29, 2019. Additional details for the connection system 100 shown in FIGS. 1 and 2 are set forth below.

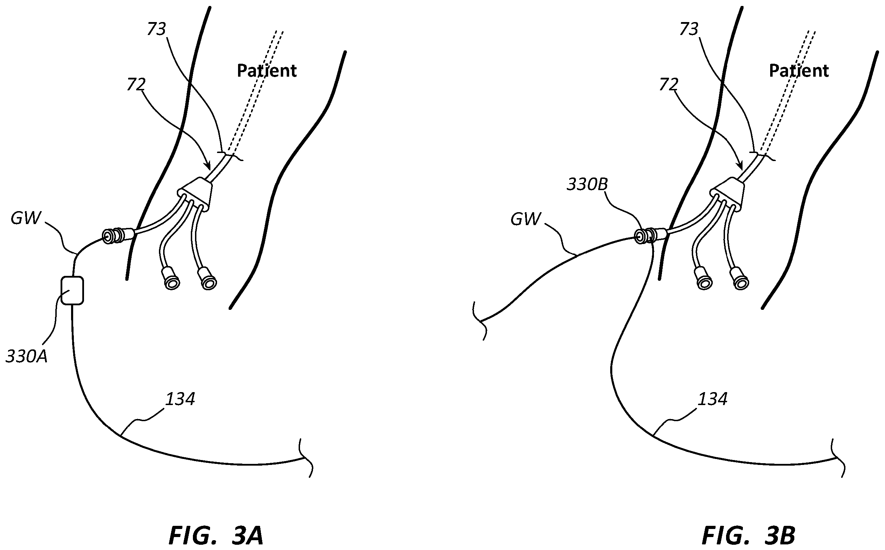

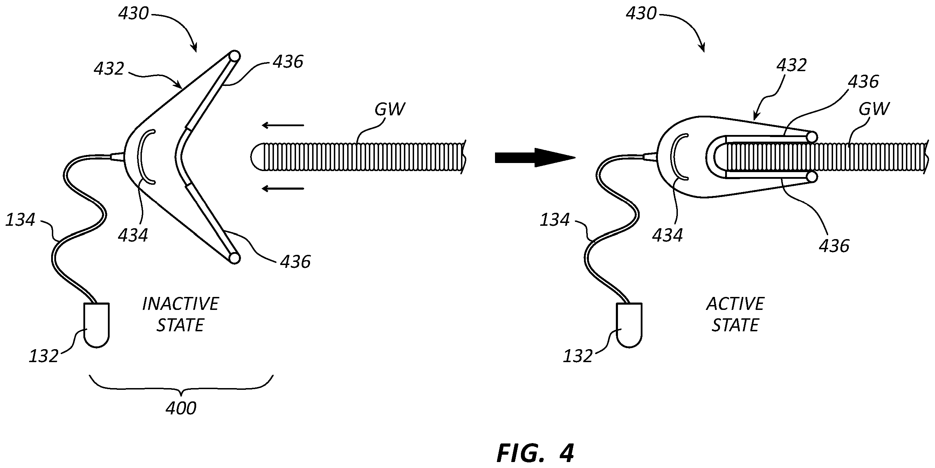

FIG. 3A illustrates the connection system 100 in accordance with a first type of connection system in accordance with some embodiments. In the first type of connection system, the probe connector 130 is separate from the system to which the probe connector 130 connects for establishing an electrical connection across a sterile field. For example, FIG. 3A shows a probe connector 330A separate from the catheter 72 but connected to the guidewire, which guidewire is removably disposed in a lumen of the catheter 72. Without enumerating each embodiment of the first type of connection system, FIG. 4 provides an example of the first type of connection system.

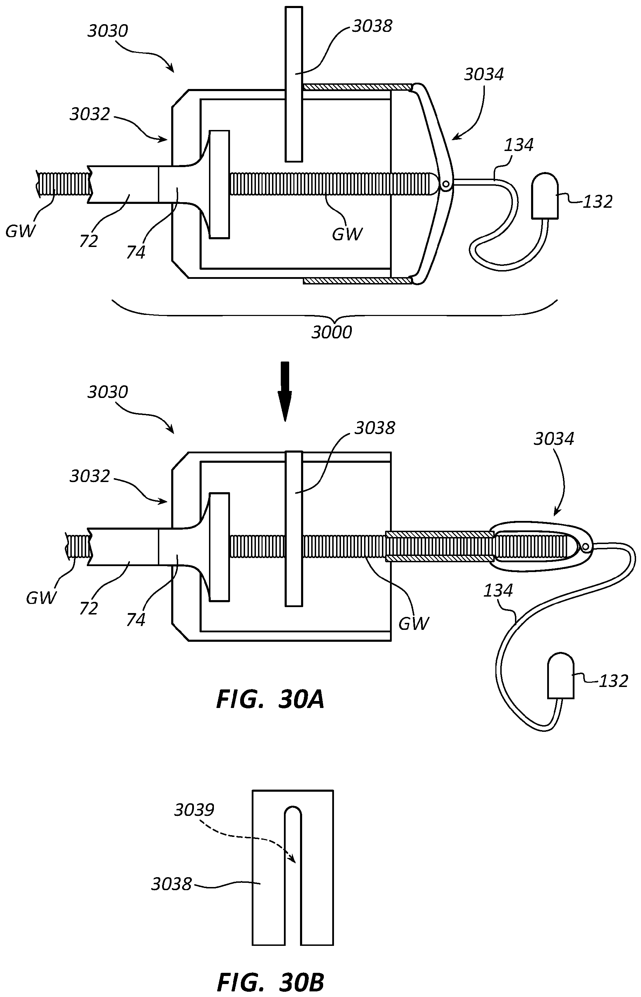

FIG. 3B illustrates the connection system 100 in accordance with a second type of connection system in accordance with some embodiments. In the second type of connection system, the probe connector 130 is integrated with the system to which the probe connector 130 connects for establishing an electrical connection across a sterile field. For example, FIG. 3B shows a probe connector 330B integrated with a hub of the catheter 72 and connected to the guidewire. Again, the guidewire is removably disposed in a lumen of the catheter 72. Without enumerating each embodiment of the second type of connection system, FIGS. 30A and 30B provide an example of the second type of connection system.

The probe connector 130, 330A, or 330B can include a probe-connecting means configured for a user to establish an electrical connection between an electrical contact of the probe connector 130, 330A, or 330B and the wire probe. The probe-connecting means includes, but is not limited to a clamp, a threadable substrate, an actuatable device, a self-actuatable device, or a threaded substrate, each of which is set forth in more detail below. Some probe-connecting means include a combination of a clamp, a threadable substrate, an actuatable device, a self-actuatable device, or a threaded substrate. With respect to clamps, a clamp of the clamps set forth below can be a directional clamp, a semi-directional clamp, or a non-directional clamp. A directional clamp generally favors a particular orientation of the wire probe to the directional clamp on account of the directional clamp's clamping mechanism, layout of electrical contacts, or the like. Often, the particular orientation is favored by a housing of the directional clamp into which the wire probe inserted. A semi-directional clamp has a limited number of orientations of the wire probe to the semi-directional clamp on account of the semi-directional probe's clamping mechanism, layout of electrical contacts, or the like. Of the limited number of orientations, a particular orientation of the wire probe to the semi-directional clamp might be favored due to the clamping mechanism, layout of electrical contacts, or the like. While a non-directional clamp might have an increased contact area with the wire probe in one or more orientations of the wire probe to the non-directional clamp, the orientation of the wire probe to the non-directional clamp is generally unlimited - excepting, for example, an orientation obstructed by a hinge of the non-directional clamp.

FIG. 4 illustrates a first connection system 400 including a first probe connector 430 in accordance with some embodiments. As shown, the probe connector 430 includes a non-directional clamp 432 configured to clamp a wire probe in any user-chosen orientation of a nearly unlimited number of orientations of the wire probe to the clamp 432. The clamp 432 includes a spring mechanism 434 having a spring such as a torsion spring that is torqued when a mouth of the clamp 432 is open and relaxed when the mouth of the clamp 432 is closed. A handle extending away from each jaw of the mouth of the clamp 432 can be incorporated into the clamp 432 to open the mouth of the clamp 432. The probe connector 430 is formed of one or more metals including alloys, one or more polymers including co-polymers or polymer blends, or a combination thereof. When formed of one or more polymers, the clamp 432 includes one or more electrical contacts 436 disposed in at least one jaw of the clamp 432, wherein the one or more electrical contacts 436 are electrically coupled to one or more wires of the tether 134.

The clamp 432 can be alternatively configured as a directional clamp. In such embodiments, the clamp 432 includes a trigger mechanism activated by inserting the wire probe into a back of the mouth of the clamp 432 with the orientation of the wire probe as shown in FIG. 4 until the wire probe engages a trigger (e.g., a plate, a pad, or a button). Upon the wire probe engaging the trigger, the mouth of the clamp 432 closes around the wire probe, thereby relaxing the spring of the spring mechanism 434. A clamping force of the mouth of the clamp 432 on the wire probe when the mouth of the clamp 432 is closed on the wire probe secures the wire probe in the mouth of the clamp 432 while providing an electrical connection.

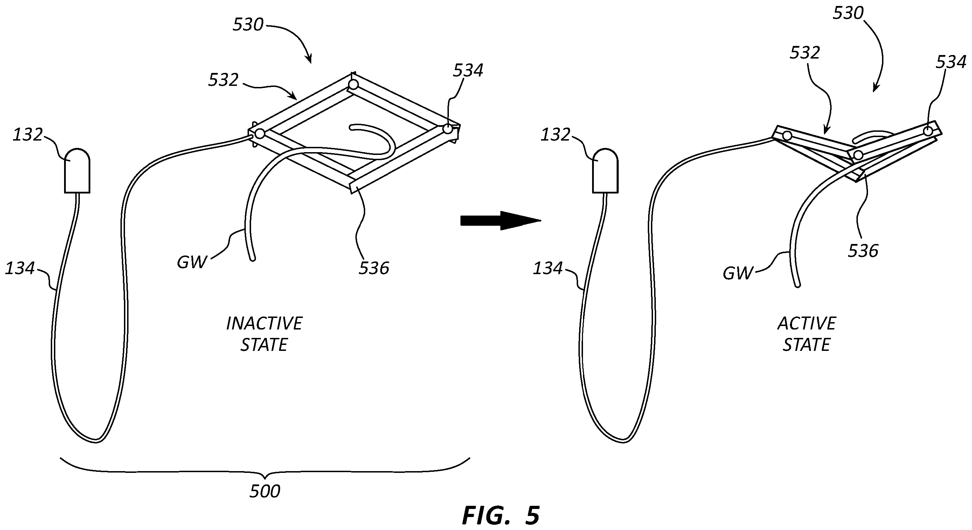

FIG. 5 illustrates a second connection system 500 including a second probe connector 530 in accordance with some embodiments. As shown, the probe connector 530 includes a semi-directional clamp 532 configured to clamp a wire probe in any user-chosen orientation of a limited number of orientations of the wire probe to the clamp 532. The limited number of orientations of the wire probe to the clamp 532 result from the wire probe having to be passed through a mouth of the clamp 532 such as orthogonal to the mouth of the clamp 532; however, the wire probe can also be passed through the mouth of the clamp 532 at other angles to the mouth of the clamp 532, thereby providing the limited number of orientations of the wire probe to the semi-directional clamp. In addition, the wire probe can be passed back through the mouth of the clamp 532 such as by wrapping the wire probe around a jaw of the clamp 532, thereby providing additional but still limited orientations of the wire probe to the clamp 532.

The clamp 532 is formed of a spring metal or alloy such as spring steel. The mouth of the clamp 532 includes hinge-pinned hinges 534 between three jaws of the mouth, as well as a living hinge 536 between two jaws of the mouth. The combination of the hinges 534 and the living hinge 536 enable the mouth of the clamp 532 to close around the wire probe after insertion of the wire probe in the mouth of the clamp 532. Closing the mouth of the clamp 532 around the wire probe tensilely deforms the spring metal of the living hinge 536 until the mouth of the clamp 532 passes an inflection point where the living hinge 536 is maximally deformed. After passing the inflection point, the spring metal of the living hinge 536 relaxes, thereby clamping the wire probe in the mouth of the clamp 532. A clamping force of the mouth of the clamp 532 on the wire probe when the mouth of the clamp 532 is closed on the wire probe secures the wire probe in the mouth of the clamp 532 while providing an electrical connection.

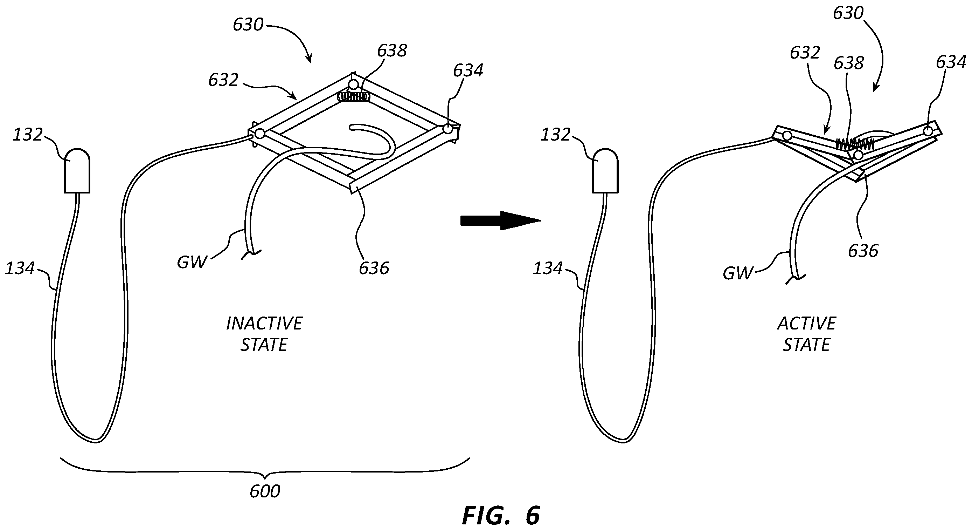

FIG. 6 illustrates a third connection system 600 including a third probe connector 630 in accordance with some embodiments. As shown, the probe connector 630 includes a semi-directional clamp 632 configured to clamp a wire probe in any user-chosen orientation of a limited number of orientations of the wire probe to the clamp 632. Like the clamp 532 of FIG. 5, the clamp 632 is formed of a spring metal or alloy such as spring steel, and a mouth of the clamp 632 includes hinge-pinned hinges 634 between three jaws of the mouth, as well as a living hinge 636 between two jaws of the mouth. In addition, the clamp 632 includes a spring mechanism 638 having a spring such as a tension spring or extension spring. The combination of the hinges 534, the living hinge 536, and the spring mechanism 638 enable the mouth of the clamp 632 to close around the wire probe after insertion of the wire probe in the mouth of the clamp 632. Closing the mouth of the clamp 632 around the wire probe tensilely deforms the spring metal of the living hinge 636 and the spring of the spring mechanism 638 until the mouth of the clamp 632 passes an inflection point where the living hinge 636 and the spring are maximally deformed. After passing the inflection point, both the spring metal of the living hinge 536 and the spring of the spring mechanism 638 relax, thereby clamping the wire probe in the mouth of the clamp 632. A clamping force of the mouth of the clamp 632 on the wire probe when the mouth of the clamp 632 is closed on the wire probe secures the wire probe in the mouth of the clamp 632 while providing an electrical connection.

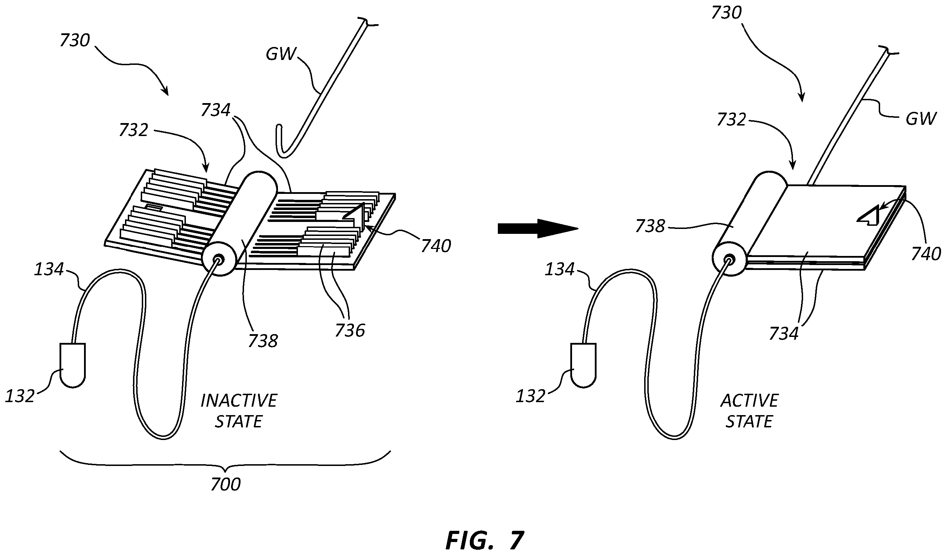

FIG. 7 illustrates a fourth connection system 700 including a fourth probe connector 730 in accordance with some embodiments. As shown, the probe connector 730 includes a semi-directional clamp 732 configured to clamp a wire probe in any user-chosen orientation of a limited number of orientations of the wire probe to the clamp 732. The limited number of orientations of the wire probe to the clamp 732 result from criteria for the wire probe having to be clamped between a pair of plates 734, as well as across one or more electrical contacts 736 disposed on each plate of the pair of plates 734. However, the limited number of orientation of wire probe to the clamp 732 include a range of orientations for the wire probe that satisfy the foregoing criteria. In addition, the wire probe can be doubled through the clamp 732, thereby providing additional but still limited orientations of the wire probe to the clamp 732.

The probe connector 730 is formed of one or more metals including alloys, one or more polymers including co-polymers or polymer blends, or a combination thereof. For example, the pair of plates 734 and a cylindrical casing 738 of a spring mechanism can be formed of the one or more polymers while the one or more electrical contacts 736 and a torsion spring (not shown) disposed within the casing 738 can be formed of the one or more metals. The torsion spring of the spring mechanism is configured to keep the clamp 732 open with the pair of plates 734 spaced apart for insertion of the wire probe between the pair of plates734. A latch 740 is configured to keep the clamp 732 latched with the pair of plates 734 pressed together for sandwiching the wire probe after the wire probe is inserted and closed in the clamp 732. If needed, the latch 740 is also configured to unlatch for repositioning the wire probe to improve an electrical connection before the wire probe is again closed in the clamp 732. A clamping force of the clamp 732 on the wire probe when the clamp 732 is closed on the wire probe secures the wire probe in the clamp 732 while providing the electrical connection to the one or more wires of the tether 134 through the one or more electrical contacts 736 of the pair of plates 734.

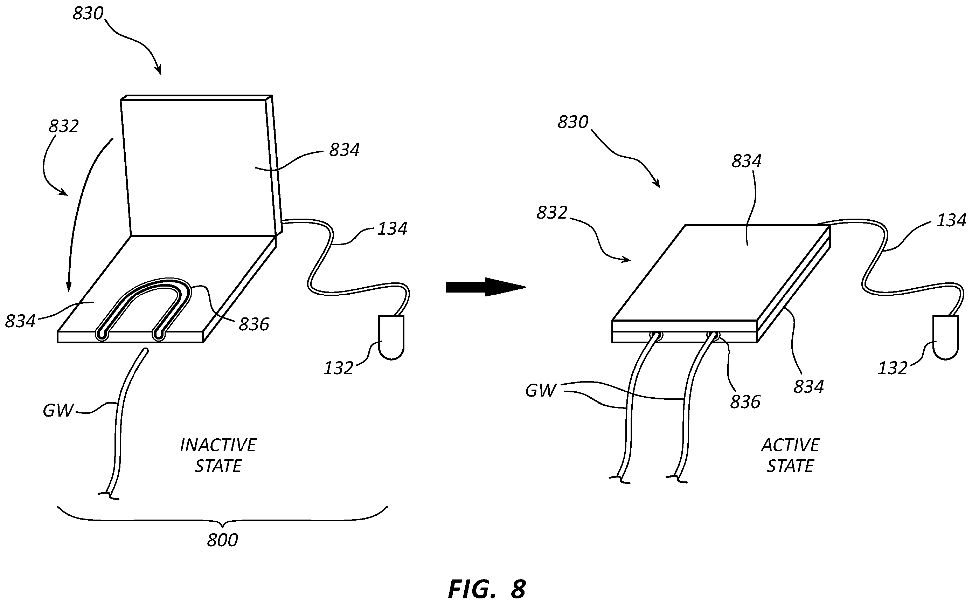

FIG. 8 illustrates a fifth connection system 800 including a fifth probe connector 830 in accordance with some embodiments. As shown, the probe connector 830 includes a directional clamp 832 configured to clamp a wire probe in a particular orientation of the wire probe to the clamp 832. The probe connector 830 is formed of one or more metals including alloys, one or more polymers including co-polymers or polymer blends, or a combination thereof. For example, a pair of plates 834 of the clamp 832 can be formed of the one or more polymers while a depression 836 contoured for the wire probe can be formed of the one or more metals to provide an electrical contact for the wire probe. The pair of plates 834 can be hinged plates coupled by a hinge pin configured to close like a book, or the pair of plates 834 can be coupled by way of spring mechanism like that of the probe connector 730. Alternatively, the spring mechanism in the latter embodiment can be configured such that the torsion spring disposed within the cylindrical casing is configured to keep the clamp 832 closed instead of open. In any of foregoing embodiments, the wire probe can be fed into the depression 836 when the clamp 832 is closed, or the wire probe can be disposed in the depression 836 when the clamp 832 is open followed by closing the clamp 832. A torturous path of the wire probe through the depression 836, a clamping force of the clamp 832 on the wire probe when the clamp 832 is closed on the wire probe, or a combination thereof secures the wire probe in the clamp 832 while providing an electrical connection to the one or more wires of the tether 134 through the depression 836 in the pair of plates 834.

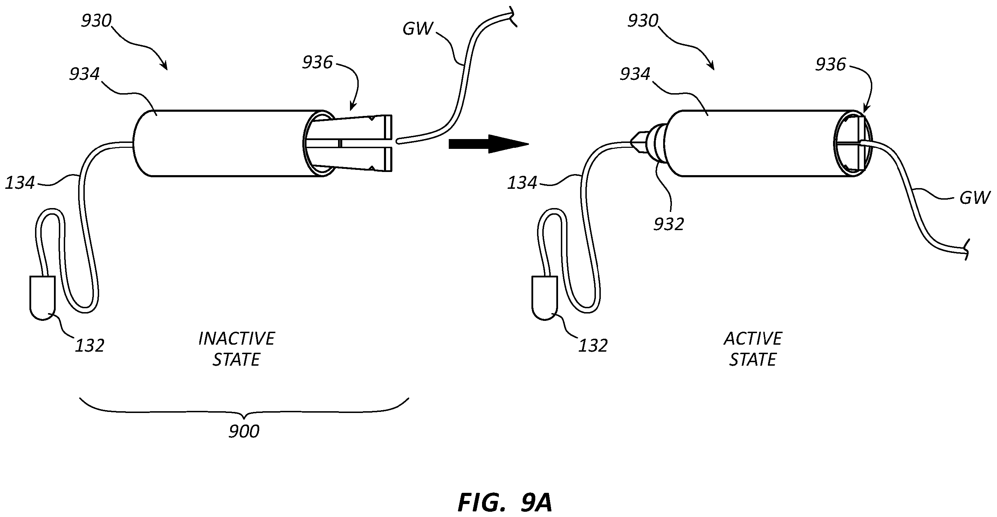

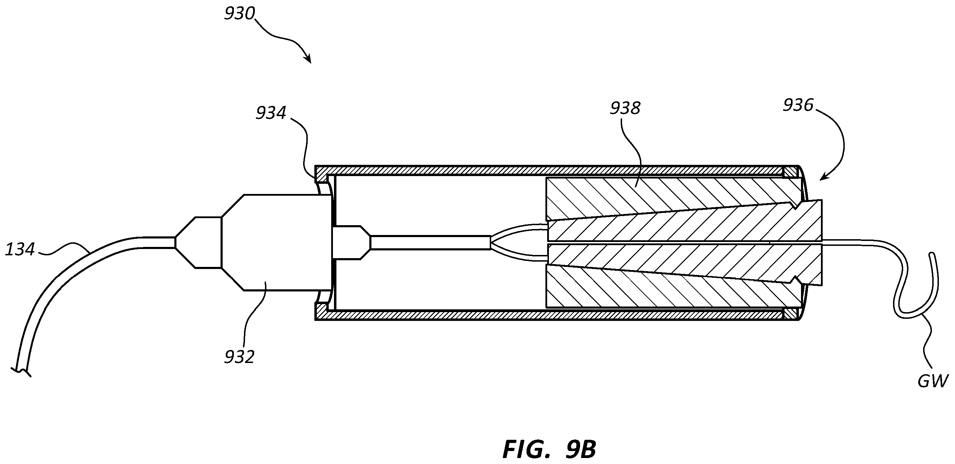

FIG. 9A illustrates a sixth connection system 900 including a sixth probe connector 930 in accordance with some embodiments. FIG. 9B illustrates a longitudinal cross section of the sixth probe connector 930 in accordance with some embodiments. As shown, the probe connector 930 includes a slideable insert 932 disposed in a housing 934, wherein the slideable insert 932 includes a tapered or conical clamp 936 coupled thereto by wires or ribbons forming a directional clamp. The housing 934 includes a fixed insert 938 in a distal end portion of the housing 934 held in the distal end portion by way of an interference fit or a bond (e.g., adhesive bond, solvent bond, etc.). Alternatively, the fixed insert 938 can be molded together with the housing 934 if the housing 934 and the fixed insert 938 are formed of one or more polymers. The fixed insert 938 includes a tapered or conical bore complementary to the tapered or conical clamp 936.

In an open-clamp position of the probe connector 930 or the slideable insert 932, which is designated as the inactive state in FIG. 9A, a proximal end portion of the slideable insert 932 is in an advanced position at or near the distal end portion of the housing 934 such that the clamp 936 substantially extends past the distal end of the housing 934. Also, in the open-clamp position, the clamp 936 is open due to mechanically biased wires or ribbons upon which jaws of the clamp 936 are mounted no longer being restricted from opening by the bore of the fixed insert 938 through which the wires or ribbons extend. A wire probe can be inserted in the clamp 936 when the clamp 936 is open, and the probe connector 930 or the slideable insert 932 can be returned to a closed-clamp position, which is designated as the active state in FIG. 9B. In the closed-clamp position, the proximal end portion of the slideable insert 932 is in an unadvanced or withdrawn position such that the clamp 936 is substantially concealed within the distal end of the housing 934. Also, in the closed-clamp position, the clamp 936 is closed due to the jaws of the clamp 936 being restricted from opening by the bore of the fixed insert 938. A latch of one or more protrusions in the fixed insert 938 corresponding to one or more wells in the clamp 936 is configured to keep the probe connector 930 or slideable insert 932 in the closed-clamp position absent any user action to return the probe connector 930 or slideable insert 932 to the open-clamp position. Such a latch keeps a wire probe in the clamp 936 when the clamp is closed on the wire probe, thereby preventing a wire-probe embolism.

The probe connector 930 can be formed of one or more metals including alloys, one or more polymers including co-polymers or polymer blends, or a combination thereof. For example, the slideable insert 932, the housing 934, the fixed insert 938, and the clamp 936 can be formed of one or more polymers while the wires or ribbons upon which the jaws of the clamp 936 are mounted can be formed of one or more metals. If the jaws of the clamp 936 are formed of the one or more polymers, one or more electrical contacts formed of the one or more metals can be disposed in the jaws of the clamp 936. The electrical contacts disposed in the jaws of the clamp 936 can be coupled to the wires or ribbons upon which the jaws of the clamp 936 are mounted, which wires or ribbons, in turn, can be coupled to the one or more wires of the tether 134 such that the wire probe forms an electrical connection to the one or more wires of the tether 134 when the wire probe is closed in the clamp 936.

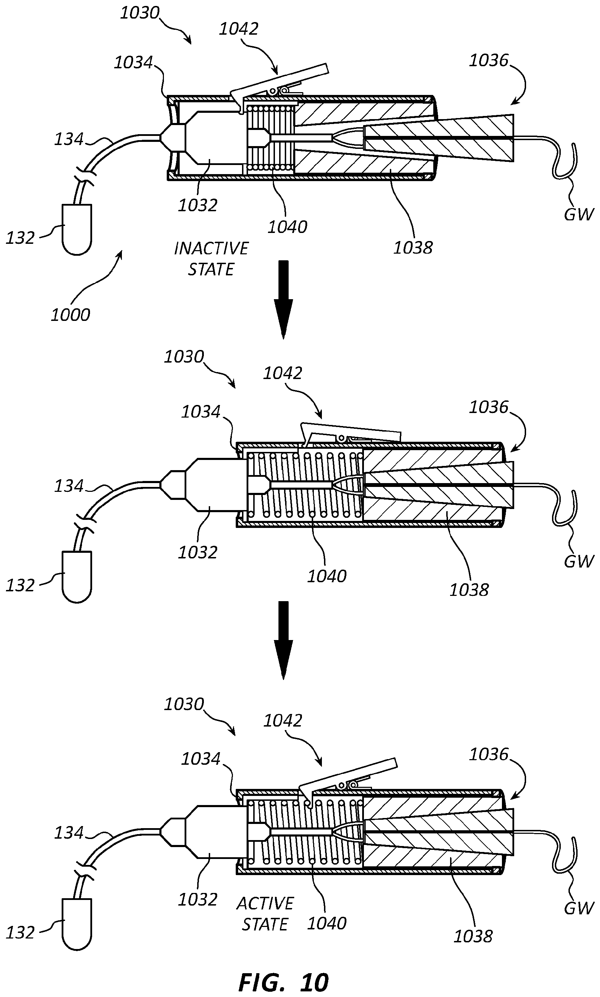

FIG. 10 illustrates a seventh connection system 1000 including a seventh probe connector 1030 in accordance with some embodiments. Like the probe connector 930, the probe connector 1030 includes a slideable insert 1032 disposed in a housing 1034, wherein the slideable insert 1032 includes a tapered or conical clamp 1036 coupled thereto by wires or ribbons forming a directional clamp. The housing 1034 includes a fixed insert 1038 in a distal end portion of the housing 1034. The fixed insert 1038 includes a tapered or conical bore complementary to the tapered or conical clamp 1036. Different than the probe connector 930, the probe connector 1030 includes a compression spring 1040 disposed between the slideable insert 1032 and the fixed insert 1038. In addition, the probe connector 1020 includes a latch 1042 with a latch arm configured to protrude through the housing 1034 and engage a shoulder of the slideable insert 1032 in an open-clamp position and a catch in a side of the slideable insert 1032 in a closed-clamp position.

In the open-clamp position of the probe connector 1030 or the slideable insert 1032, which is designated as the inactive state in FIG. 10, a proximal end portion of the slideable insert 1032 is in an advanced position at or near the distal end portion of the housing 1034 such that the clamp 1036 substantially extends past the distal end of the housing 1034. As shown, the compression spring 1040 is compressed and the slideable insert 1032 is held in the advanced position by the latch 1042, wherein the latch arm protrudes through the housing 1034 and engages the shoulder of the slideable insert 1032. The clamp 1036 is open when the slideable insert 1032 is in the advanced position due to mechanically biased wires or ribbons upon which jaws of the clamp 1036 are mounted no longer being restricted from opening by the bore of the fixed insert 1038 through which the wires or ribbons extend. A wire probe can be inserted in the clamp 1036 when the clamp 1036 is open, and the probe connector 1030 or the slideable insert 1032 can be returned to the closed-clamp position by disengaging the latch 1042. In the closed-clamp position, which is designated as the active state in FIG. 10, the proximal end portion of the slideable insert 1032 is in an unadvanced or withdrawn position such that the clamp 1036 is substantially concealed within the distal end of the housing 1034. As shown, the compression spring 1040 is relaxed and the slideable insert 1032 is held in the unadvanced or withdrawn position by the latch 1042, wherein the latch arm protrudes through the housing 1034 and engages the catch in the side of the slideable insert 1032. The clamp 1036 is closed when the slideable insert 1032 is in the unadvanced or withdrawn position due to the jaws of the clamp 1036 being restricted from opening by the bore of the fixed insert 1038. Like the of the probe connector 930, the latch 1042 keeps a wire probe in the clamp 1036 when the clamp is closed on the wire probe, thereby preventing a wire-probe embolism.

The probe connector 1030 can be formed of one or more metals including alloys, one or more polymers including co-polymers or polymer blends, or a combination thereof. For example, the slideable insert 1032, the housing 1034, the fixed insert 1038, and the clamp 1036 can be formed of one or more polymers while the wires or ribbons upon which the jaws of the clamp 1036 are mounted can be formed of one or more metals. If the jaws of the clamp 1036 are formed of the one or more polymers, one or more electrical contacts formed of the one or more metals can be disposed in the jaws of the clamp 1036. The electrical contacts disposed in the jaws of the clamp 1036 can be coupled to the wires or ribbons upon which the jaws of the clamp 1036 are mounted, which wires or ribbons, in turn, can be coupled to the one or more wires of the tether 134 such that the wire probe forms an electrical connection to the one or more wires of the tether 134 when the wire probe is closed in the clamp 1036.

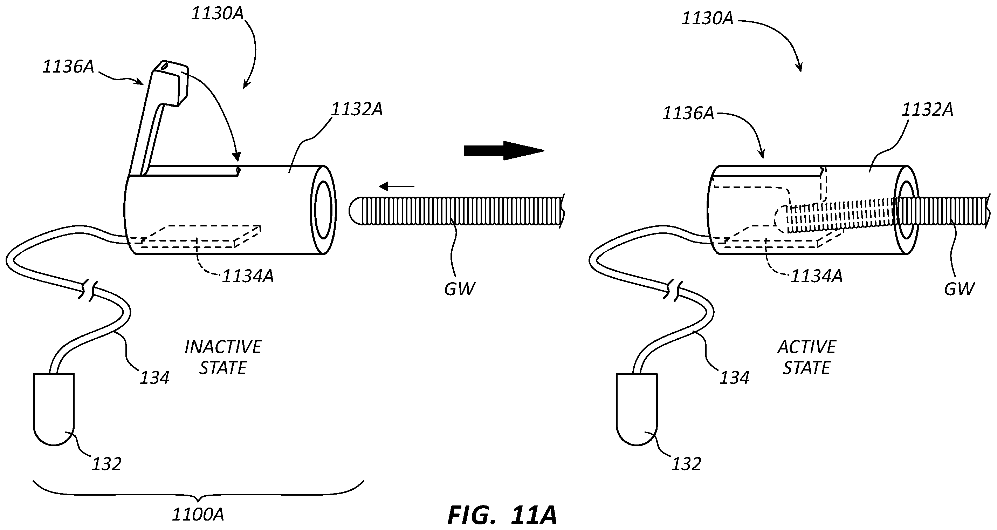

FIG. 11A illustrates an eighth connection system 1100A including an eighth probe connector 1130A in accordance with some embodiments. As shown, the probe connector 1130A includes a housing 1132A, one or more electrical contacts 1134A disposed within the housing 1132A on an inner surface of the housing 1132A, and a clamp 1136A integrated into a side of the housing 1132A opposite the one or more electrical contacts 1134A forming a directional clamp. The clamp 1136A is joined to the side of the housing 1132A by a living hinge between the clamp 1136A and the side of the housing 1132A. The probe connector 1130A includes an open-clamp position with a jaw of the clamp 1136A substantially extending out from the side of the housing 1134A. A wire probe can be inserted into the housing 1132A when the probe connector 1130A is in the open-clamp position. Advantageously, a user can view a location of the wire probe in the housing 1134A when the probe connector 1130A is in the open-clamp position since the clamp 1136A provides a window in the side of the housing 1134A when open. The probe connector 1130A also includes a closed-clamp position with the jaw of the clamp 1136A substantially extending into the housing 1132A adjacent the one or more electrical contacts 1134A or against the one or more electrical contacts 1134A. When the wire probe is present in the probe connector 1130A while in the closed-clamp position, the jaw of the clamp 1136A clamps the wire probe against the one or more electrical contacts 1134A with a sufficient clamping force to secure the wire probe in the probe connector 1130A.

The probe connector 1130A can be formed of one or more metals including alloys, one or more polymers including co-polymers or polymer blends, or a combination thereof. For example, the housing 1132A and the clamp 1136A can be formed of the one or more polymers while the one or more electrical contacts 1134A can be formed of the one or more metals. The one or more electrical contacts 1134A can be coupled to the one or more wires of the tether 134 such that the wire probe forms an electrical connection to the one or more wires of the tether 134 when the wire probe is clamped by the clamp 1136A.

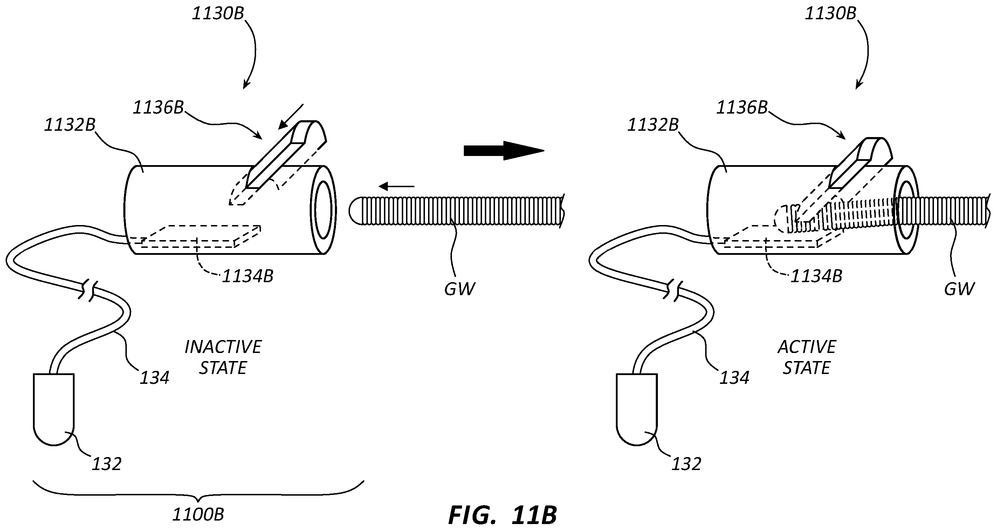

FIG. 11B illustrates a ninth connection system 1100B including a ninth probe connector 1130B in accordance with some embodiments. As shown, the probe connector 1130B includes a housing 1132B, one or more electrical contacts 1134B disposed within the housing 1132B on an inner surface of the housing 1132B, and a clamp 1136B integrated into a side of the housing opposite the one or more electrical contacts 1134B forming a directional clamp. The clamp 1136B includes a plate disposed in an angled slot in the side of the housing 1132B such that the plate can slide in or out of the housing 1132B depending upon whether the probe connector 1130B, or the clamp 1136B thereof, is in an open-clamp position or a closed-clamp position. The open-clamp position has the clamp 1136B substantially extending out from the side of the housing 1134B. A wire probe can be inserted into the housing 1132B when the probe connector 1130B is in the open-clamp position. The closed-clamp position has the clamp 1136B substantially extending into the housing 1132B adjacent the one or more electrical contacts 1134B or against the one or more electrical contacts 1134B. When the wire probe is present in the probe connector 1130B while in the closed-clamp position, the clamp 1136B clamps the wire probe against the one or more electrical contacts 1134B with a sufficient clamping force to secure the wire probe in the probe connector 1130B. A latch (not shown) can be configured to keep the clamp 1136B latched together with the housing 1132B to keep the wire probe in the probe connector 113B, thereby preventing a wire-probe embolism.

The probe connector 1130B can be formed of one or more metals including alloys, one or more polymers including co-polymers or polymer blends, or a combination thereof. For example, the housing 1132B and the clamp 1136B can be formed of the one or more polymers while the one or more electrical contacts 1134B can be formed of the one or more metals. The one or more electrical contacts 1134B can be coupled to the one or more wires of the tether 134 such that the wire probe forms an electrical connection to the one or more wires of the tether 134 when the wire probe is clamped by the clamp 1136B.

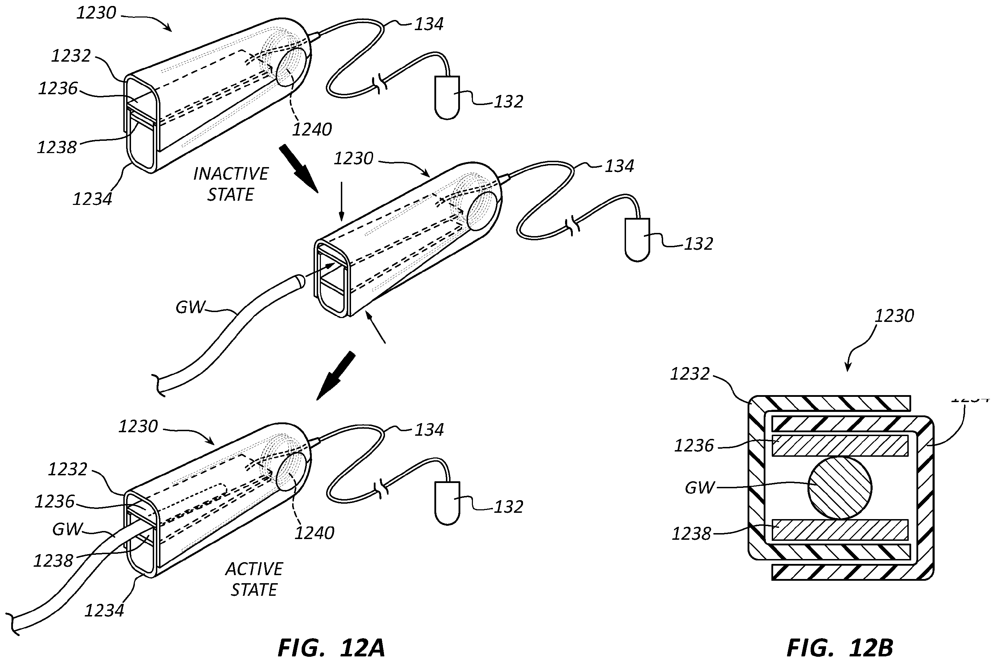

FIG. 12A illustrates a tenth connection system 1200 including a tenth probe connector 1230 in accordance with some embodiments. FIG. 12B illustrates a transverse cross section of the tenth probe connector 1230 in accordance with some embodiments.

As shown, the probe connector 1230 includes a housing including a top portion of the housing 1232 and a bottom portion of the housing 1234, one or more electrical contacts such as an electrical contact 1236 and an electrical contact 1238 disposed within the housing, and a spring 1240 at a proximal end of the probe connector 1230 forming a directional clamp. The top portion of the housing 1232 is 'C' shaped and the bottom portion of the housing 1234 is ' 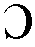 ' shaped such that the top portion of the housing 1232 interlocks with the bottom portion of the housing 1234. The spring 1240 at the proximal end of the probe connector 1230 can be a torsion spring that is torqued when the top portion of the housing 1232 and the bottom portion of the housing 1234 are pushed together (e.g., by squeezing the probe connector 1230 about a distal end portion of the probe connector 1230), thereby opening a mouth of the clamp. When the spring 1240 is allowed to relax (e.g., by releasing the probe connector 1230), the top portion of the housing 1232 and the bottom portion separate from each other to close the mouth of the clamp. At least one electrical contact of the electrical contacts 1236 and 1238 is disposed on a mating surface of the top portion of the housing 1232 that mates with the bottom portion of the housing 1234 or a mating surface of the bottom portion of the housing 1234 that mates with the top portion of the housing 1232. In the embodiment shown in FIGS. 12A and 12B, each mating surface of the top portion of the housing 1232 and the bottom portion of the housing 1234 includes an electrical contact. As best shown in FIG. 12B, the mating surface of the top portion of the housing 1232 includes the electrical contact 1238 and the bottom portion of the housing 1234 includes the electrical contact 1236.

In view of the probe connector 1230 shown in FIGS. 12A and 12B, which includes two electrical contacts, a wire probe can be inserted into the housing between the electrical contacts 1236 and 1238 when the probe connector 1230 is in an open-clamp position with the top portion of the housing 1232 and the bottom portion of the housing 1234 pushed together with a compressive force. Once the wire probe is disposed within the housing, the compressive force can be removed, thereby returning the probe connector 1230 to a closed-clamp position in which the mating surfaces of the top portion of the housing 1232 and the bottom portion of the housing 1234 are mated with the wire probe between the electrical contacts 1236 and 1238. The probe connector 1230 clamps the wire probe against the electrical contacts 1236 and 1238 with a sufficient clamping force to secure the wire probe in the probe connector 1230.

The probe connector 1230 can be formed of one or more metals including alloys, one or more polymers including co-polymers or polymer blends, or a combination thereof. For example, the housing can be formed of the one or more polymers while the one or more electrical contacts such as the electrical contacts 1236 and 1238 can be formed of the one or more metals. The one or more electrical contacts can be coupled to the one or more wires of the tether 134 such that the wire probe forms an electrical connection to the one or more wires of the tether 134 when the wire probe is clamped by the probe connector 1230.

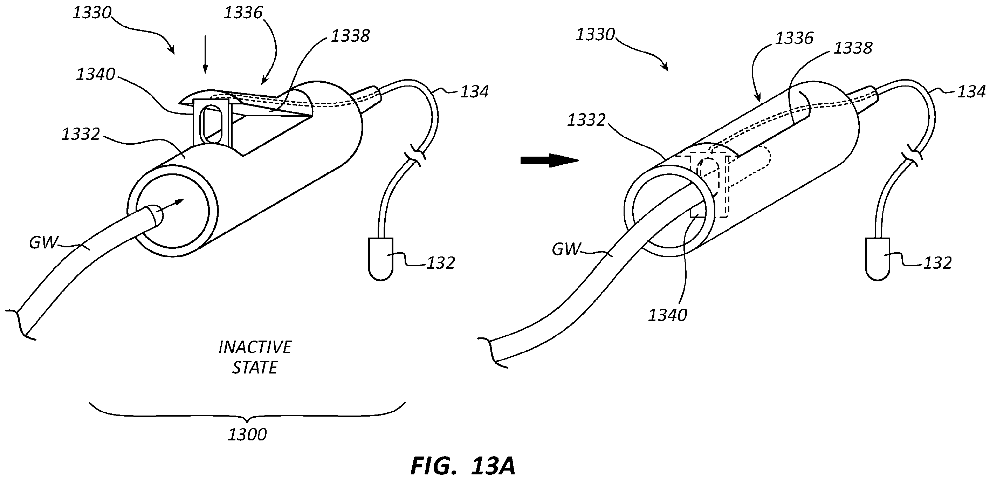

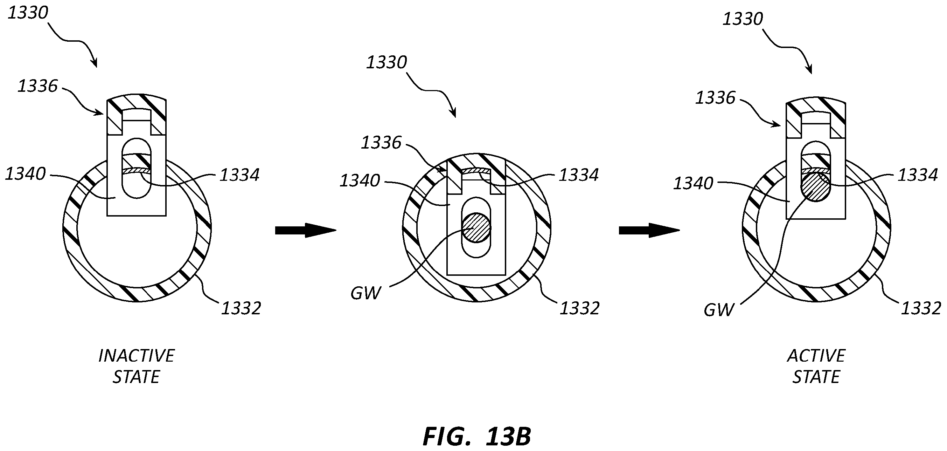

FIG. 13A illustrates an eleventh connection system 1300 including an eleventh probe connector 1330 in accordance with some embodiments. FIG. 13B illustrates a transverse cross section of the eleventh probe connector 1330 in accordance with some embodiments. As shown, the probe connector 1330 includes a housing 1332, one or more electrical contacts 1334 disposed within the housing 1332 on an inner surface of the housing 1332, and a clamp 1336 integrated or incorporated into a side of the housing 1332 on a same side of the housing 1332 as at least one electrical contact of the one or more electrical contacts 1334 forming a directional clamp. The clamp 1336 includes a mechanically biased plate 1338 cut out from or coupled to a side of a distal end portion of the housing 1332 and an eye plate 1340 orthogonally coupled to the plate 1338 having an eye configured to accept a wire probe. If the plate 1338 is cut out from the side of the housing 1336, a cutout in the housing 1332 exists for the plate 1338 and the eye plate 1340 of the clamp 1336 to move therethrough. If the plate 1338 is coupled to the side of the housing 1336, a transverse slot is cut in the side of the housing 1332 for the eye plate 1340 of the clamp 1336 to move therethrough.

In an alternative configuration of the probe connector 1330, the one or more electrical contacts 1334 on the inner surface of the housing 1332 can instead line at least a bottom of the eye of the eye plate 1340.

An open-clamp position of the probe connector 1330, or the clamp 1336 thereof, has at least the eye plate 1340 of the clamp 1336 substantially extending into the housing 1332 through the cutout in the housing 1332 or the transverse slot in the housing 1332. When the probe connector 1330 is in the open-clamp position, a wire probe can be inserted into the housing 1332 and threaded through the eye of the eye plate 1340 as a threadable substrate of the probe connector 1330. A closed-clamp position has the eye plate 1340 of the clamp 1336 substantially extending out from the side of the housing 1332 but prevented by the wire probe in the eye of the eye plate 1340 from extending as far as the mechanical bias of the plate 1338 would otherwise allow. When the wire probe is present in the probe connector 1330 while in the closed-clamp position, the clamp 1336 clamps the wire probe against the one or more electrical contacts 1334 disposed within the housing 1332 on the inner surface of the housing 1332. Alternatively, the clamp 1336 clamps the wire probe against the one or more electrical contacts 1334 lining at least the bottom of the eye of the eye plate 1340. The clamp 1336 clamps the wire probe with a sufficient clamping force to secure the wire probe in the probe connector 1330.

The probe connector 1330 can be formed of one or more metals including alloys, one or more polymers including co-polymers or polymer blends, or a combination thereof. For example, the housing 1332, the plate 1338, and the eye plate 1340 can be formed of the one or more polymers while the one or more electrical contacts 1334 can be formed of the one or more metals. The one or more electrical contacts 1334 can be coupled to the one or more wires of the tether 134 such that the wire probe forms an electrical connection to the one or more wires of the tether 134 when the wire probe is clamped by the clamp 1336.

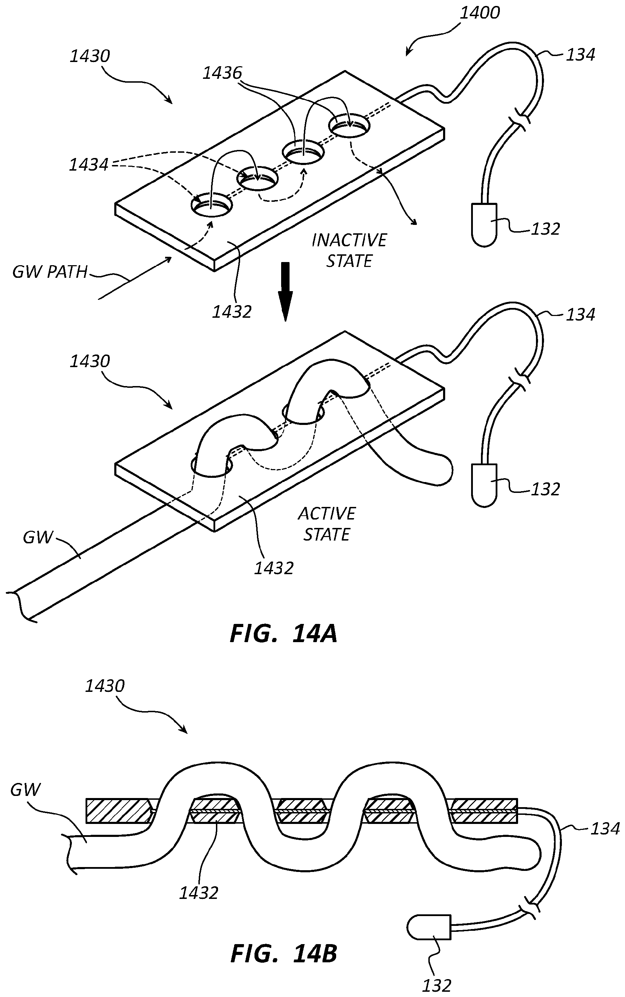

FIG. 14A illustrates a twelfth connection system 1400 including a twelfth probe connector 1430 in accordance with some embodiments. FIG. 14B illustrates a longitudinal cross section of the twelfth probe connector 1430 in accordance with some embodiments. As shown, the probe connector 1430 includes a substrate 1432, one or more through holes 1434 through the substrate 1432, and a corresponding number of electrical contacts 1436 lining the one or more through holes 1434 forming a threadable substrate of the probe connector 1430. The one or more through holes 1434 can be linearly arranged as shown in FIGS. 14A and 14B, or the one or more through holes 1434 can be arranged in any other combination (e.g., a square). More through holes 1434 such as two through holes 1434, three through holes 1434, or the four through holes 1434 shown in FIGS. 14A and 14B generally provide a more torturous path through the substrate 1432.

A wire probe can be threaded through any one or more through holes 1434 of the substrate 1432 in any combination thereof. Threading the wire probe through all the through holes 1434 generally provides the most torturous path of the wire probe through the substrate 1432. Once the wire probe is threaded through the one or more through holes 1434 of the substrate 1432, the wire probe makes sufficient contact with the electrical contacts 1436, and the wire probe is sufficiently secured in place in the probe connector 1330, thereby preventing a wire-probe embolism.

The probe connector 1430 can be formed of one or more metals including alloys, one or more polymers including co-polymers or polymer blends, or a combination thereof. For example, the substrate 1432 can be formed of the one or more polymers while the electrical contacts 1436 can be formed of the one or more metals. The electrical contacts 1436 can be coupled to the one or more wires of the tether 134 such that the wire probe forms an electrical connection to the one or more wires of the tether 134 when the wire probe is secured in the probe connector 1330.

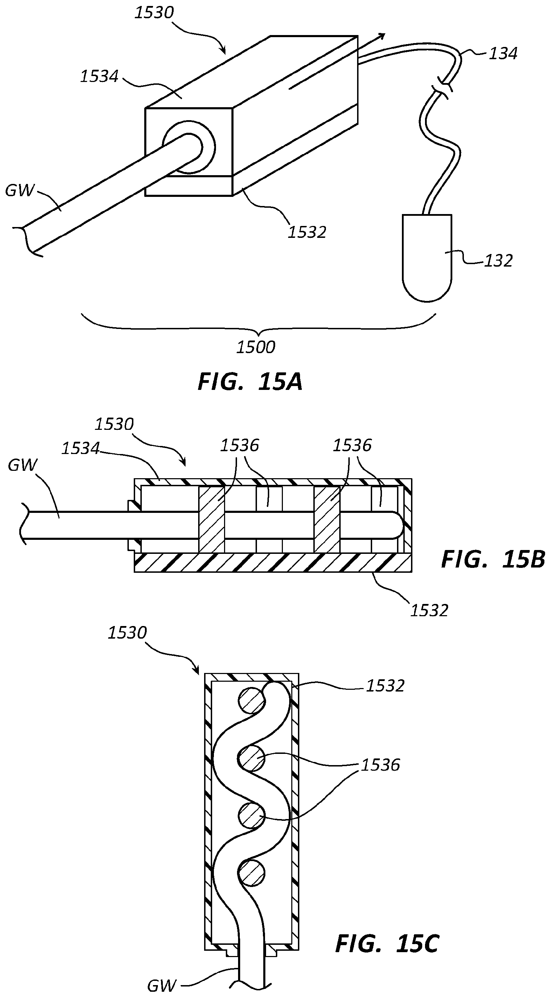

FIG. 15A illustrates a thirteenth connection system 1500 including a thirteenth probe connector 1530 in accordance with some embodiments. FIG. 15B illustrates a transverse cross section of the thirteenth probe connector 1530 in accordance with some embodiments. FIG. 15C illustrates a longitudinal cross section of the thirteenth probe connector 1530 in accordance with some embodiments. As shown, the probe connector 1530 includes a substrate 1532, a housing 1534 over the substrate 1532, and one or more poles 1536 disposed on the substrate 1532 forming a threadable substrate of the probe connector 1530. The housing 1534 incudes a through hole in at least a distal end of the housing 1534, and the housing 1534 can be configured to slide onto or off the substrate 1532 as needed for threading a wire probe through the one or more poles 1536, checking the wire probe, or removing the wire probe from the probe connector 1530. The probe connector 1530 also includes one or more electrical contacts (not shown) corresponding to the one or more poles 1536, which one or more electrical contacts line the one or more poles 1536. The one or more poles 1536 can be linearly or staggeredly arranged as shown in FIGS. 15B and 15C, or the one or more poles 1536 can be arranged in any other combination (e.g., a square). More poles 1536 such as two poles 1536, three poles 1536, or the four poles 1536 shown in FIGS. 15B and 15C generally provide a more torturous path through the probe connector 1530.

A wire probe can be threaded through any one or more poles 1536 disposed on the substrate 1532 in any combination thereof. Threading the wire probe through all the poles 1536 generally provides the most torturous path of the wire probe through the probe connector 1530. Once the wire probe is threaded through the one or more poles 1536 dispose on the substrate 1532, the wire probe makes sufficient contact with the one or more electrical contacts lining the one or more poles 1536, and the wire probe is sufficiently secured in place in the probe connector 1530, thereby preventing a wire-probe embolism.

The probe connector 1530 can be formed of one or more metals including alloys, one or more polymers including co-polymers or polymer blends, or a combination thereof. For example, the substrate 1532, the housing 1534, and the one or more poles 1536 can be formed of the one or more polymers while the one or more electrical contacts can be formed of the one or more metals. The one or more electrical contacts can be coupled to the one or more wires of the tether 134 such that the wire probe forms an electrical connection to the one or more wires of the tether 134 when the wire probe is secured in the probe connector 1530.

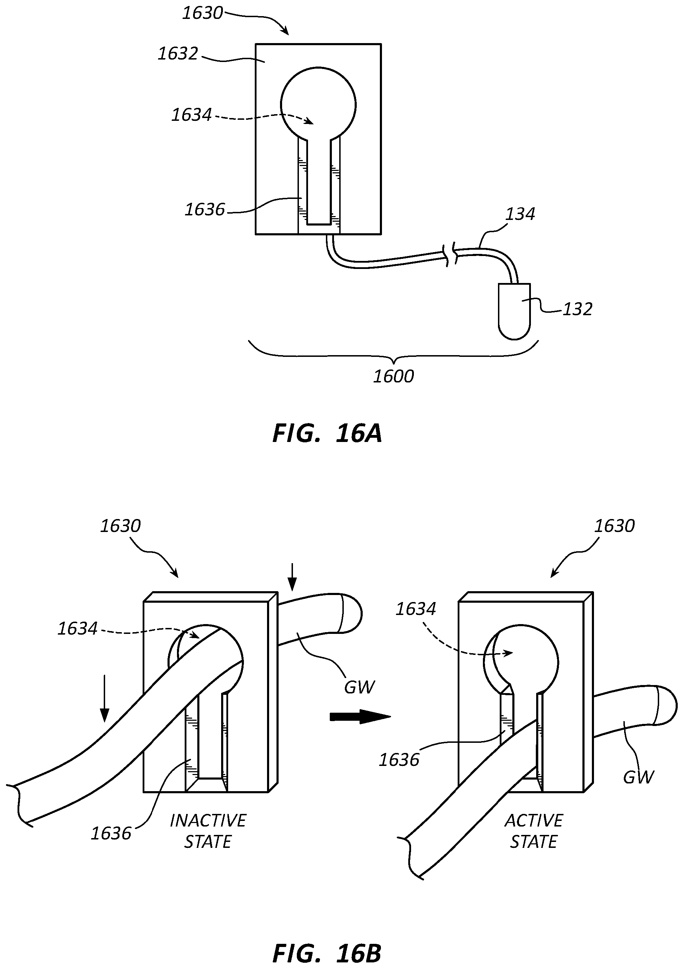

FIG. 16A illustrates a fourteenth connection system 1600 including a fourteenth probe connector 1630 in accordance with some embodiments. FIG. 16B illustrates the fourteenth connection system 1600 with a wire probe in accordance with some embodiments.

As shown, the probe connector 1630 includes a substrate 1632, a cutout 1634 in the substrate 1632, and one or more electrical contacts 1634 lining at least a portion of the substrate 1632 around the cutout 1634 forming a threadable substrate of the probe connector 1630. The substrate 1632 can be a plate, and the cutout 1634 can have a keyhole shape formed of a relatively wider circular portion of the cutout 1634 joined to a relatively narrower rectangular portion of the cutout 1634 as shown. The one or more electrical contacts 1634 can line the rectangular portion of the cutout 1634 or the cutout 1634 in its entirety.

A wire probe can be threaded through the relatively wider circular portion of the cutout 1634 and subsequently inserted or pushed into the relatively narrower rectangular portion of the cutout 1634. Once the wire probe is inserted into the rectangular portion of the cutout 1634, the wire probe makes sufficient contact with the one or more electrical contacts 1634 lining at least the rectangular portion of the cutout 1634, and the wire probe is sufficiently secured by friction in place in the probe connector 1630, thereby preventing a wire-probe embolism.

The probe connector 1630 can be formed of one or more metals including alloys, one or more polymers including co-polymers or polymer blends, or a combination thereof. For example, the substrate 1632 can be formed of the one or more polymers while the one or more electrical contacts 1636 can be formed of the one or more metals. The one or more electrical contacts 1636 can be coupled to the one or more wires of the tether 134 such that the wire probe forms an electrical connection to the one or more wires of the tether 134 when the wire probe is secured in the probe connector 1630.

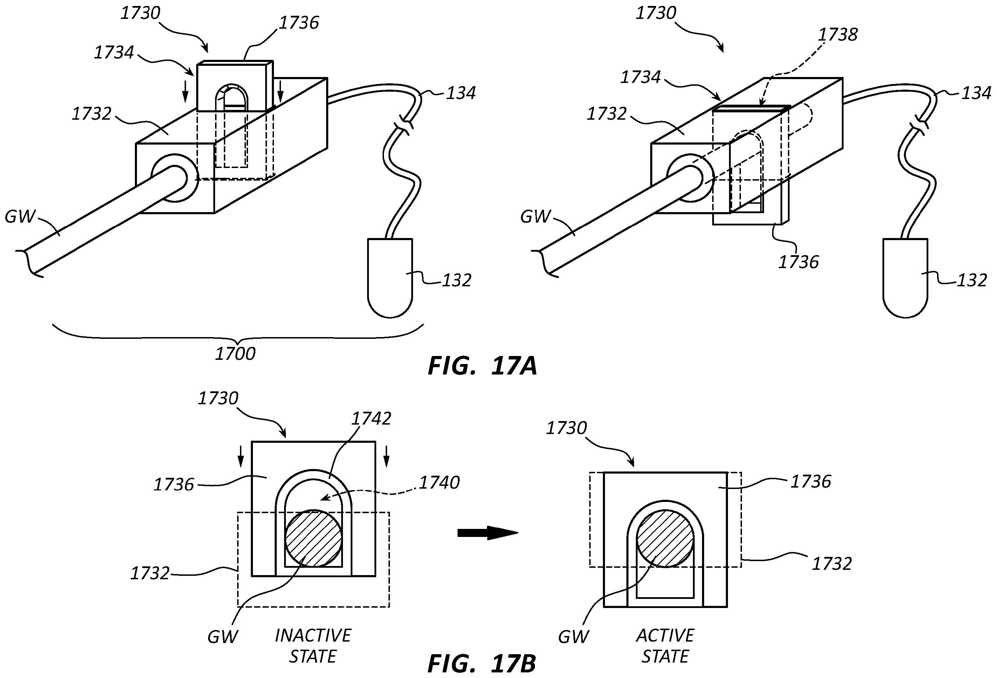

FIG. 17A illustrates a fifteenth connection system 1700 including a fifteenth probe connector 1730 in accordance with some embodiments. FIG. 17B illustrates a transverse cross section of the fifteenth probe connector 1730 in accordance with some embodiments.

As shown, the probe connector 1730 includes a housing 1732 and a clamp 1734 integrated into the housing 1732 forming a directional clamp. The clamp 1734 includes a plate 1736 configured for disposal through a slot 1738 extending through both a side of the housing 1732 and an opposite side of the housing 1732 such that the plate 1736 can slide in or out of the housing 1732. The plate 1736 includes a cutout 1740, which can have the threadable keyhole shape of the cutout 1634 of the substrate 1632 of the probe connector 1630 or a rounded rectangle shape, or arch shape, as shown in FIG. 17B. The probe connector 1730 includes one or more electrical contacts 1742 lining at least a portion of the plate 1736 around the cutout 1740.

An open-clamp position of the probe connector 1730 has the plate 1736 of the clamp 1736 substantially extending out from the side of the housing 1732. When the probe connector 1730 is in the open-clamp position, a wire probe can be inserted into the housing 1732 and threaded through the cutout 1740 as a threadable substrate of the probe connector 1730. A closed-clamp position has the plate 1736 of the clamp 1734 substantially extending into the housing 1732 and at least partially through the slot 1738 through the opposite side of the housing 1732. The plate 1736 is prevented from extending completely through the slot 1738 of the housing 1732 by the wire probe in the cutout 1740 of the plate 1736. When the wire probe is present in the probe connector 1730 while in the closed-clamp position, the wire probe is clamped against the one or more electrical contacts 1742 with a sufficient clamping force to secure the wire probe in the probe connector 1730.

The probe connector 1730 can be formed of one or more metals including alloys, one or more polymers including co-polymers or polymer blends, or a combination thereof. For example, the housing 1732 and the plate 1736 of the clamp 1734 can be formed of the one or more polymers while the one or more electrical contacts 1742 can be formed of the one or more metals. The one or more electrical contacts 1742 can be coupled to the one or more wires of the tether 134 such that the wire probe forms an electrical connection to the one or more wires of the tether 134 when the wire probe is clamped by the clamp 1734.

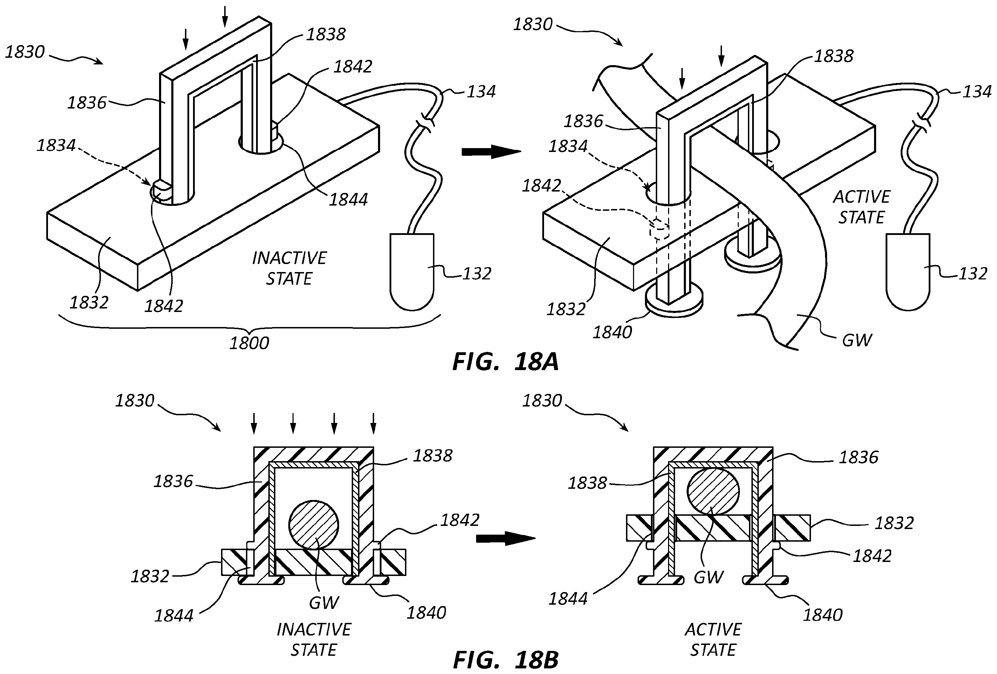

FIG. 18A illustrates a sixteenth connection system 1800 including a sixteenth probe connector 1830 in accordance with some embodiments. FIG. 18B illustrates a transverse cross section of the sixteenth probe connector 1830 in accordance with some embodiments. As shown, the probe connector 1830 includes a substrate 1832, a pair of through holes 1834 through the substrate 1832, a staple 1836 having two legs disposed in the through holes 1834, one or more electrical contacts 1838 lining at least a portion of a mating side of the staple 1836 that mates with a mating surface of the substrate 1832. The staple 1836 includes a pair of feet 1840 with each foot at an end of a leg of the staple 1836. The pair of feet 1840 are configured to keep the staple 1836 captively disposed in the substrate 1832. The staple 1836 also includes a pair of latching protrusions 1842 with each leg of the staple 1836 having one latching protrusion of the pair of latching protrusions 1842. The pair of latching protrusions 1842 is part of a latching mechanism also including a pair of catches 1844 in the substrate 1832, wherein one catch is formed in a side of each through hole of the pair of through holes 1834. The two legs of the staple 1836 can be mechanically biased away from each other to facilitate latching the pair of latching protrusions 1842 and the pair of catchers 1844.

A wire probe can be threaded between the two legs of the staple 1838, as well as between the staple and the substrate 1832, which provides a threadable substrate for the probe connector 1830; however, the probe connector 1830 also includes a semi-directional clamp by way of the staple 1836, the substrate 1832, and the latching mechanism therebetween. An open-clamp position of the probe connector 1830 has the staple 1836 substantially extending out from the substrate 1832. A wire probe can be threaded in any user-desired fashion between at least the two legs of the staple 1838, as well as between the staple 1836 and the substrate 1832, when the probe connector 1830 is in the open-clamp position. A closed-clamp position of the probe connector 1830 has the staple 1836 substantially extending through the substrate 1832. The staple 1836 is also latched to the substrate 1832 by way of the latching mechanism in the closed-clamp position preventing the staple 1836 from returning to the open-clamp position without user action. When the wire probe is present in the probe connector 1830 while in the closed-clamp position, the wire probe is clamped against the one or more electrical contacts 1738 with a sufficient clamping force to secure the wire probe in the probe connector 1830.

The probe connector 1830 can be formed of one or more metals including alloys, one or more polymers including co-polymers or polymer blends, or a combination thereof. For example, the substrate 1832 and the staple 1836 can be formed of the one or more polymers while the one or more electrical contacts 1838 can be formed of the one or more metals. The one or more electrical contacts 1838 can be coupled to the one or more wires of the tether 134 such that the wire probe forms an electrical connection to the one or more wires of the tether 134 when the wire probe is secured in the probe connector 1330.

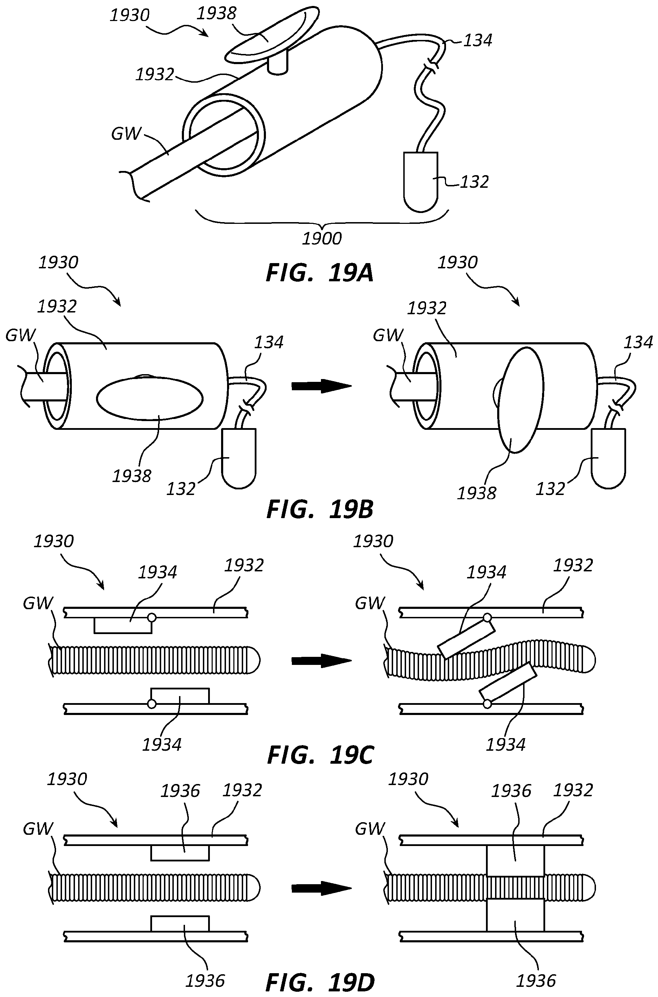

FIG. 19A illustrates a first view of a seventeenth connection system 1900 including a seventeenth probe connector 1930 in accordance with some embodiments. FIG. 19B illustrates a second view of the seventeenth connection system 1900 including the seventeenth probe connector 1930 in accordance with some embodiments. FIG. 19C illustrates a connecting mechanism of the seventeenth probe connector 1730 in accordance with some embodiments. FIG. 19D illustrates an alternative connecting mechanism of the seventeenth probe connector 1930 in accordance with some embodiments. As shown, the probe connector 1930 includes a housing 1932 having an opening in a distal end of the housing 1932, one or more electrical contacts 1934 *(see* FIG. 19C) or 1936 *(see* FIG. 19D) disposed within the housing 1932 on opposite sides of an inner surface of the housing 1332, and an actuator 1938 such as a knob or handle integrated or incorporated into a side of the housing 1932 forming a user-actuatable probe connector 1930. The actuator 1938 is configured to turn through an angle to actuate a connecting mechanism and establish both a mechanical and an electrical connection with a wire probe inserted in the distal end of the housing 1932. As shown in FIG. 19C, the connecting mechanism simultaneously swings each hinge-mounted electrical contact of the one or more electrical contacts 1934 toward a longitudinal axis of the probe connector 1930 such as by a suitably configured connecting wire or rod coupled to the actuator 1938. As shown in FIG. 19D, the connecting mechanism simultaneously advances each wall-mounted electrical contact of the one or more electrical contacts 1936 toward a longitudinal axis of the probe connector 1930 such as by a suitably configured connecting wire or rod coupled to the actuator 1938.

When the probe connector 1930 is in an inactive state before the actuator 1938 actuates the connecting mechanism, a wire probe can be inserted into the distal end of the housing 1932 between the one or more electrical contacts 1934 *(see* FIG. 19C) or 1936 *(see* FIG. 19D). When the probe connector 1930 is in an active state after the actuator 1938 actuates the connecting mechanism, the wire probe is sandwiched between the one or more electrical contacts 1934 *(see* FIG. 19C) or 1936 *(see* FIG. 19D) within the housing 1932. When the wire probe is present in the probe connector 1930 while in the active state, the one or more electrical contacts 1934 *(see* FIG. 19C) or 1936 *(see* FIG. 19D) clamp the wire probe between the one or more electrical contacts 1934 or 1936 with a sufficient clamping force to secure the wire probe in the probe connector 1930 and provide an electrical connection to the tether 134.

The probe connector 1930 can be formed of one or more metals including alloys, one or more polymers including co-polymers or polymer blends, or a combination thereof. For example, the housing 1932 and the actuator 1938 can be formed of the one or more polymers while the one or more electrical contacts 1934 or 1936 and conducting parts of the connecting mechanism can be formed of the one or more metals. The one or more electrical contacts 1934 or 1936 can be coupled to the one or more wires of the tether 134 such that the wire probe forms an electrical connection to the one or more wires of the tether 134 when the wire probe is clamped by the one or more electrical contacts 1934 or 1936.

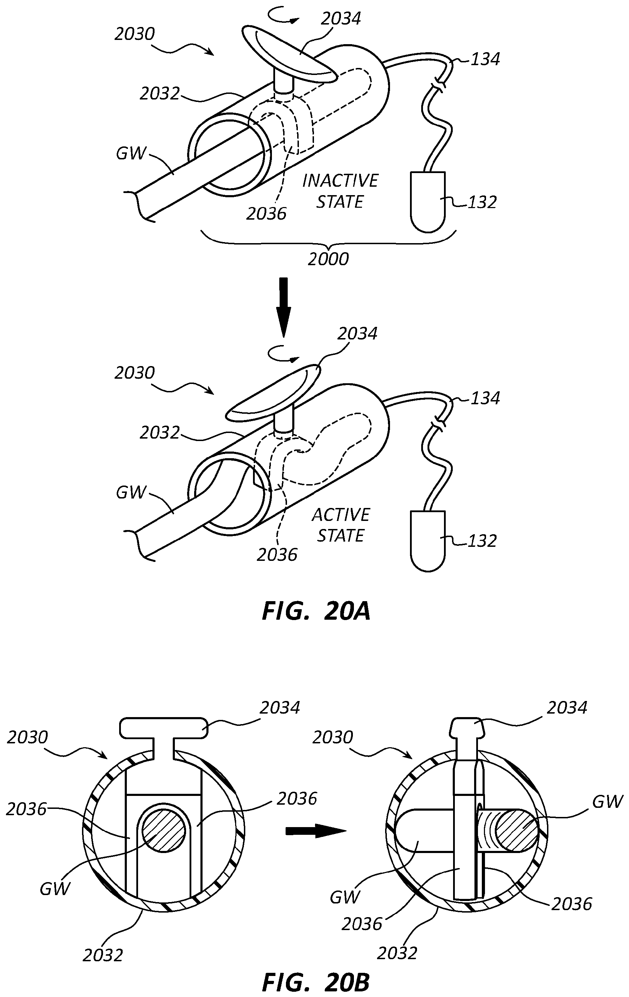

FIG. 20A illustrates an eighteenth connection system 2000 including an eighteenth probe connector 2030 in accordance with some embodiments. FIG. 20B illustrates a transverse cross section of the eighteenth probe connector 2030 in accordance with some embodiments. As shown, the probe connector 2030 includes a housing 2032 having an opening in a distal end of the housing 2032, an actuator 2034 such as a knob or handle integrated or incorporated into a side of the housing 2032, and two electrical contacts 2036 such as two prongs extending from the actuator 2034, or linings over the two prongs of the actuator 2034, forming a user-actuatable probe connector 2030. The actuator 2034 is configured to turn through an angle to actuate a connecting mechanism and establish both a mechanical and an electrical connection with a wire probe inserted in the distal end of the housing 2032. As shown in FIG. 20B, since the two prongs including the two electrical contacts 2036 extend from the actuator 2034, the two prongs including the electrical contacts 2036 rotate through the same angle as the actuator 2034 when the connecting mechanism is activated by rotating the actuator 2034.

When the probe connector 2030 is in an inactive state before the actuator 2034 actuates the connecting mechanism, a wire probe can be inserted into the distal end of the housing 2032 between the two electrical contacts 2036. When the probe connector 2030 is in an active state after the actuator 2034 actuates the connecting mechanism, the wire probe is tortuously disposed or slightly twisted between the two electrical contacts 2036 within the housing 2032. When the wire probe is present in the probe connector 2030 while in the active state, the two electrical contacts 2036 hold the wire probe between the two electrical contacts 2036 with a sufficient frictional force to secure the wire probe in the probe connector 2030 and provide an electrical connection to the tether 134.

The probe connector 2030 can be formed of one or more metals including alloys, one or more polymers including co-polymers or polymer blends, or a combination thereof. For example, the housing 2032 and at least a portion of the actuator 2038 such as a core of the actuator 2038 or the knob or handle portion thereof can be formed of the one or more polymers while the two electrical contacts 2036 can be formed of the one or more metals. The two electrical contacts 2036 can be coupled to the one or more wires of the tether 134 such that the wire probe forms an electrical connection to the one or more wires of the tether 134 when the wire probe is disposed between the two electrical contacts 2036.

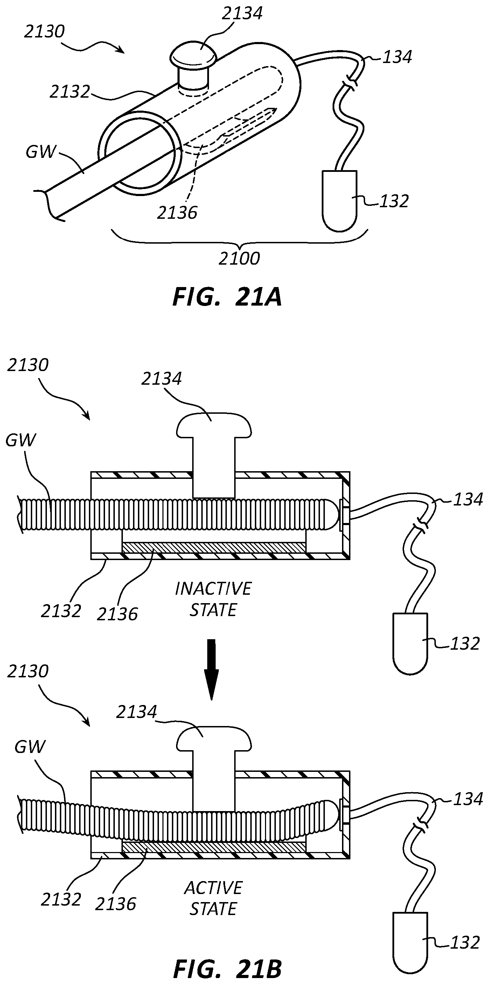

FIG. 21A illustrates a nineteenth connection system 2100 including a nineteenth probe connector 2130 in accordance with some embodiments. FIG. 21B illustrates a longitudinal cross section of the nineteenth probe connector 2130 in accordance with some embodiments. As shown, the probe connector 2130 includes a housing 2132 having an opening in a distal end of the housing 2132, an actuator 2134 such as a push button integrated or incorporated into a side of the housing 2132, and one or more electrical contacts 2136 *(see* FIG. 21B) disposed on an inner surface of the housing 2132 on an opposite side of the housing 2132 from the actuator 2134 forming a user-actuatable probe connector 2130. The actuator 2134, or an extension thereof such as a rubber boot, is configured to insert into the housing 2132 upon actuation in a connecting mechanism that establishes both a mechanical and an electrical connection with a wire probe inserted in the distal end of the housing 2132. As shown in FIG. 21B, the actuator 2134 is configured to lodge the wire probe against the one or more electrical contacts 2136 disposed on the inner surface of the housing 2132 on the opposite side of the housing 2132 from the actuator 2134.

When the probe connector 2130 is in an inactive state before the actuator 2134 actuates the connecting mechanism, a wire probe can be inserted into the distal end of the housing 2132 over the one or more electrical contacts 2136 *(see* FIG. 21B). When the probe connector 2130 is in an active state after the actuator 2134 actuates the connecting mechanism, the wire probe is lodged between the actuator 2134, or the extension thereof, and the one or more electrical contacts 2136 within the housing 2132 and cannot be withdrawn without depressing the actuator 2134. Thus, in the active state, the wire probe is lodged within the housing 2132 against the one or more electrical contacts 2136 with a sufficient frictional force to secure the wire probe in the probe connector 2130 and provide an electrical connection to the tether 134.

The probe connector 2130 can be formed of one or more metals including alloys, one or more polymers including co-polymers or polymer blends, or a combination thereof. For example, the housing 2132 and the actuator 2134 can be formed of the one or more polymers while the one or more electrical contacts 2136 can be formed of the one or more metals. The one or more electrical contacts 2136 can be coupled to the one or more wires of the tether 134 such that the wire probe forms an electrical connection to the one or more wires of the tether 134 when the wire probe is clamped against the one or more electrical contacts 2136 by the actuator 2134.

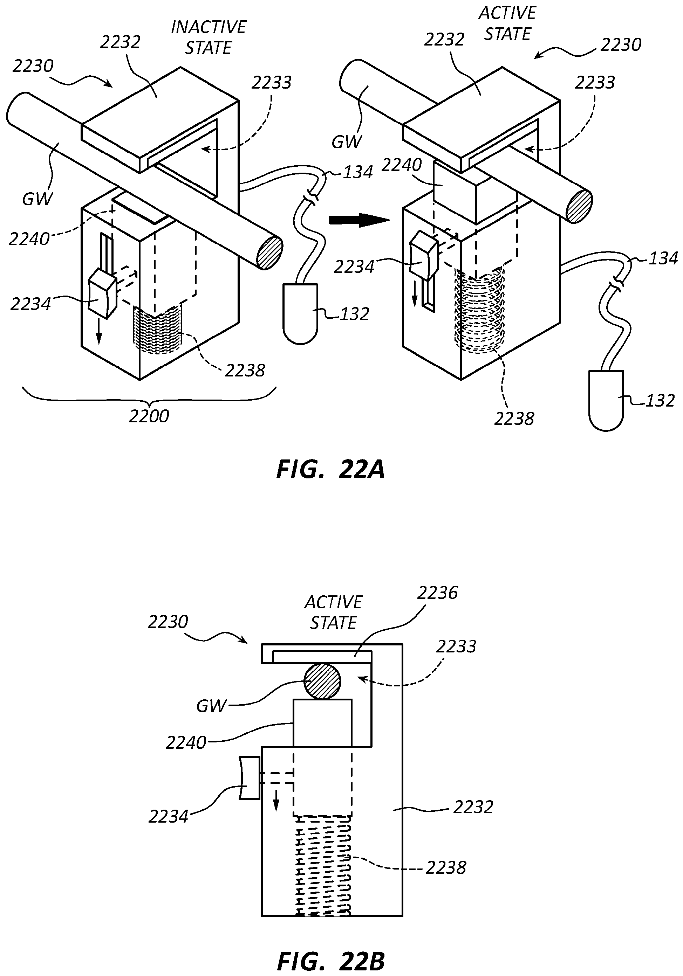

FIG. 22A illustrates a twentieth connection system 2200 including a twentieth probe connector 2230 in accordance with some embodiments. FIG. 22B illustrates a longitudinal cross section of the twentieth probe connector 2230 in accordance with some embodiments. As shown, the probe connector 2230 is a user-actuatable probe connector 2230 including a housing 2232 having a wire-probe channel 2233 in a distal end of the housing 2232, an actuator 2234 such as a slide button integrated or incorporated into a side of the housing 2232, and one or more electrical contacts 2236 *(see* FIG. 22B) disposed on an inner surface of the wire-probe channel 2233 on an opposite side of the channel 2233 from the actuator 2234. The actuator 2234 is configured to slide within an actuator channel 2235 in a side of the probe connector 2230 from a spring-loaded position to a spring-released position. In the spring-loaded position, a spring 2238 such as a compression spring is compressed and a plunger 2240 coupled to the spring 2238 is withdrawn into the housing 2232. In the spring-released position, the spring 2238 is relaxed and the plunger 2240 is extended into the wire-probe channel 2233 against the one or more electrical contacts 2236. The actuator 2234 can be toggled from the spring-loaded position to the spring-released position as needed to load or unload a wire probe from the probe connector 2230.

When the probe connector 2230 is in an inactive state and the actuator 2234 is in the spring-loaded position, a wire probe can be inserted into the wire-probe channel 2233 in the distal end of the housing 2232. Subsequent to actuating the connecting mechanism, with the probe connector 2230 in an active state and the actuator 2234 in the spring-unloaded position, the wire probe is lodged between the plunger 2240 and the one or more electrical contacts 2236 within the wire-probe channel 2233 and cannot be withdrawn without a user returning the actuator 2234 to the spring-loaded position. Thus, in the active state, the wire probe is lodged within the wire-probe channel 2233 against the one or more electrical contacts 2236 with a sufficient frictional force to secure the wire probe in the probe connector 2230 and provide an electrical connection to the tether 134.

The probe connector 2230 can be formed of one or more metals including alloys, one or more polymers including co-polymers or polymer blends, or a combination thereof. For example, the housing 2232, the actuator 2234, and the plunger 2240 can be formed of the one or more polymers while the one or more electrical contacts 2236 and the spring 2238 can be formed of the one or more metals. The one or more electrical contacts 2236 can be coupled to the one or more wires of the tether 134 such that the wire probe forms an electrical connection to the one or more wires of the tether 134 when the wire probe is clamped against the one or more electrical contacts 2236 by the plunger 2240.

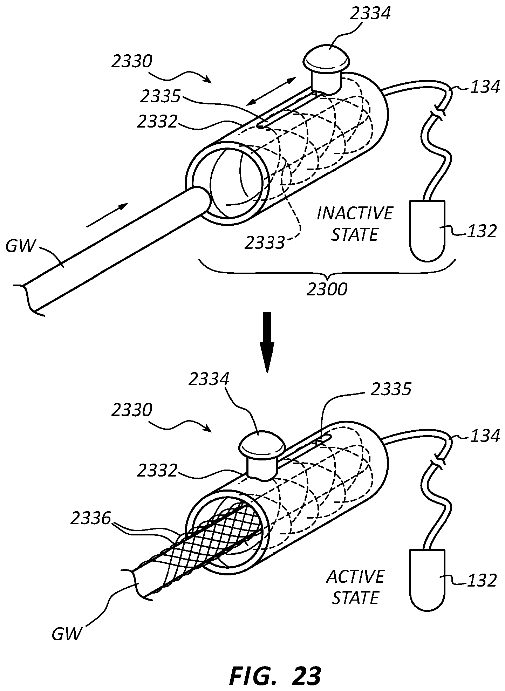

FIG. 23 illustrates a twenty-first connection system 2300 including a twenty-first probe connector 2330 in accordance with some embodiments. As shown, the probe connector 2330 includes a housing 2332 having an opening in a distal end of the housing 2332, an actuator 2334 such as a slide button configured to slide within an actuator channel 2335 in a side of the housing 2332, and electrical contacts 2336 *(see* FIG. 23, bottom) configured as conductive filaments or wires mounted on an actuator-coupled internal component disposed within the housing 2332, thereby forming a user-actuatable probe connector 2330. While not shown in FIG. 23, an internal wall of the housing 2332 tapers from a proximal end of the housing 2332 to the distal end of the housing 2332. In addition, a luminal surface of the housing 2332 includes spiral grooves 2333 that spiral from the proximal end to the distal end of the housing 2332. The spiral grooves 2333 are configured to direct the electrical contacts 2336 along the luminal surface of the housing 2332 when moved therethrough on the internal component.

When the probe connector 2330 is in an inactive state and the actuator 2334 is in a corresponding inactivated position, a wire probe can be inserted into the distal end of the housing 2332 loosely within the electrical contacts 2336, which are disposed in the spiral grooves 2333 of the housing 2332. When the probe connector 2330 is in an active state and the actuator 2334 is in a corresponding activated position, the wire probe is tightly lodged within the electrical contacts 2336 where the internal wall of the housing 2332 is most tapered. The wire probe cannot be withdrawn from the housing 2332 without sliding the actuator 2334 back into the inactivated position. Thus, in the active state, the wire probe is lodged within the housing 2332 by the electrical contacts 2336 with a sufficient frictional force to secure the wire probe in the probe connector 2330 and provide an electrical connection to the tether 134.

The probe connector 2330 can be formed of one or more metals including alloys, one or more polymers including co-polymers or polymer blends, or a combination thereof. For example, the housing 2332 and the actuator 2334 can be formed of the one or more polymers while the electrical contacts 2336 can be formed of the one or more metals. The electrical contacts 2336 can be coupled to the one or more wires of the tether 134 such that the wire probe forms an electrical connection to the one or more wires of the tether 134 when the wire probe is clamped by the electrical contacts 2336.

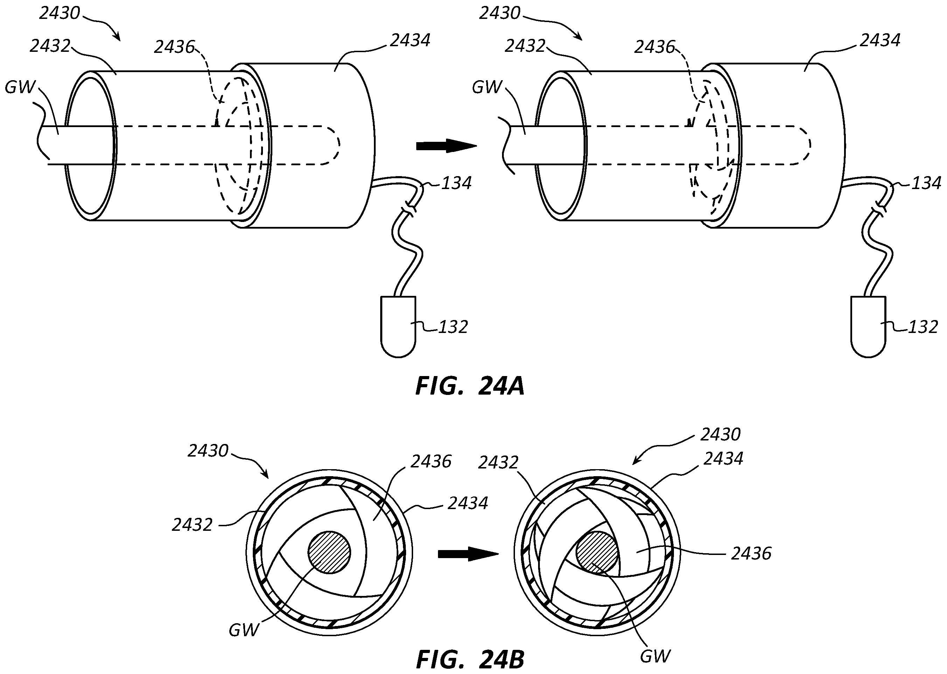

FIG. 24A illustrates a twenty-second connection system 2400 including a twenty-second probe connector 2430 in accordance with some embodiments. FIG. 24B illustrates a transverse cross section of the twenty-second probe connector 2430 in accordance with some embodiments. As shown, the probe connector 2430 includes a housing 2432 having an opening in a distal end of the housing 2432, an actuator 2434 such as a turnable proximal-end piece configured to twist or turn with respect to the housing 2432, and a diaphragmatic arrangement of blade-like electrical contacts 2436 *(see* FIG. 24B) mounted on an actuator-coupled internal component disposed within the housing 2432, thereby forming a user-actuatable probe connector 2430.

When the probe connector 2430 is in an inactive state and the actuator 2434 is in a corresponding inactivated position (e.g., the proximal-end piece is turned as far as possible in a counterclockwise direction), a wire probe can be inserted into the distal end of the housing 2432 within the arrangement of electrical contacts 2436. When the probe connector 2430 is in an active state and the actuator 2434 is in a corresponding activated position (e.g., the proximal-end piece is turned as far as possible in a clockwise direction), the wire probe is tightly lodged within the arrangement of electrical contacts 2436. The wire probe cannot be withdrawn from the housing 2432 without turning the actuator 2434 back into the inactivated position. Thus, in the active state, the wire probe is lodged within the housing 2432 by the arrangement of electrical contacts 2436 with a sufficient frictional force to secure the wire probe in the probe connector 2430 and provide an electrical connection to the tether 134.

The probe connector 2430 can be formed of one or more metals including alloys, one or more polymers including co-polymers or polymer blends, or a combination thereof. For example, the housing 2432 and the actuator 2434 can be formed of the one or more polymers while the arrangement of electrical contacts 2436 can be formed of the one or more metals. The arrangement of electrical contacts 2436 can be coupled to the one or more wires of the tether 134 such that the wire probe forms an electrical connection to the one or more wires of the tether 134 when the wire probe is clamped by the arrangement of electrical contacts 2436.

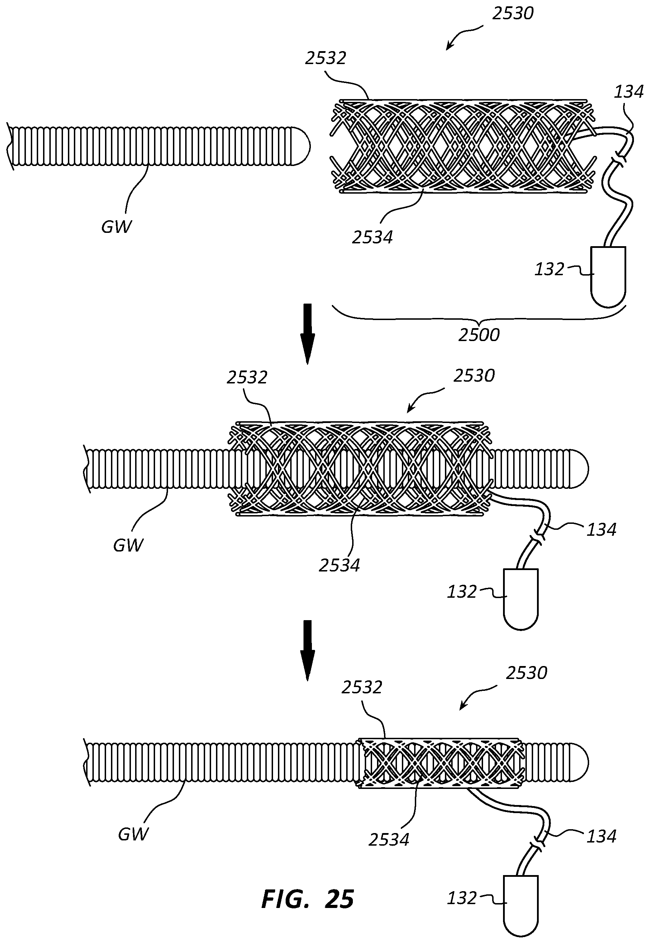

FIG. 25 illustrates a twenty-third connection system 2500 including a twenty-third probe connector 2530 in accordance with some embodiments. As shown, the probe connector 2530 is a user-actuatable probe connector 2530 including a housing 2532 having an opening in both a distal end of the housing 2532 and a proximal end of the housing 2532. The housing 2532 is braided with electrical contacts 2534 such as conducting filaments or wires forming helically wound braids of the housing 2532. In addition, an actuator of the probe connector is the housing 2532, itself, as extending a length of the housing 2532 (e.g., by pulling ends of the housing 2532 away from each other) reduces a diameter of the housing 2532 while compressing the length of the housing 2532 (e.g., by pushing the ends of the housing 2532 toward each other) increases the diameter of the housing 2532 on account of the helically wound braids of the electrical contacts 2534.

When the probe connector 2530 is in an inactive state (e.g., the ends of the housing 2532 are pushed in toward each other), a wire probe can be inserted into the distal end of the housing 2532. When the probe connector 2530 is in an active state (e.g., the ends of the housing 2532 are pulled out away from each other), the wire probe is tightly lodged within the housing 2532. The wire probe cannot be withdrawn from the housing 2532 without returning the housing 2532 back into the inactive state. Thus, in the active state, the wire probe is lodged within the housing 2532 by a reduced diameter of the housing 2532 with a sufficient frictional force to secure the wire probe in the probe connector 2530 and provide an electrical connection to the tether 134.

The probe connector 2530 can be formed of one or more metals including alloys, one or more polymers including co-polymers or polymer blends, or a combination thereof. For example, the housing 2532, which is braided with the electrical contacts 2534 such as conducting filaments or wires forming helically wound braids of the housing 2532, can be formed of the one or more metals. Some of the electrical contacts 2534 such as every other electrical contact can be coated with a polymer such as rubber to enhance the frictional force that secure the wire probe in the probe connector 2530 in the active state. The electrical contacts 2534 can be coupled to the one or more wires of the tether 134 such that the wire probe forms an electrical connection to the one or more wires of the tether 134 when the wire probe is lodged within the housing 2532.

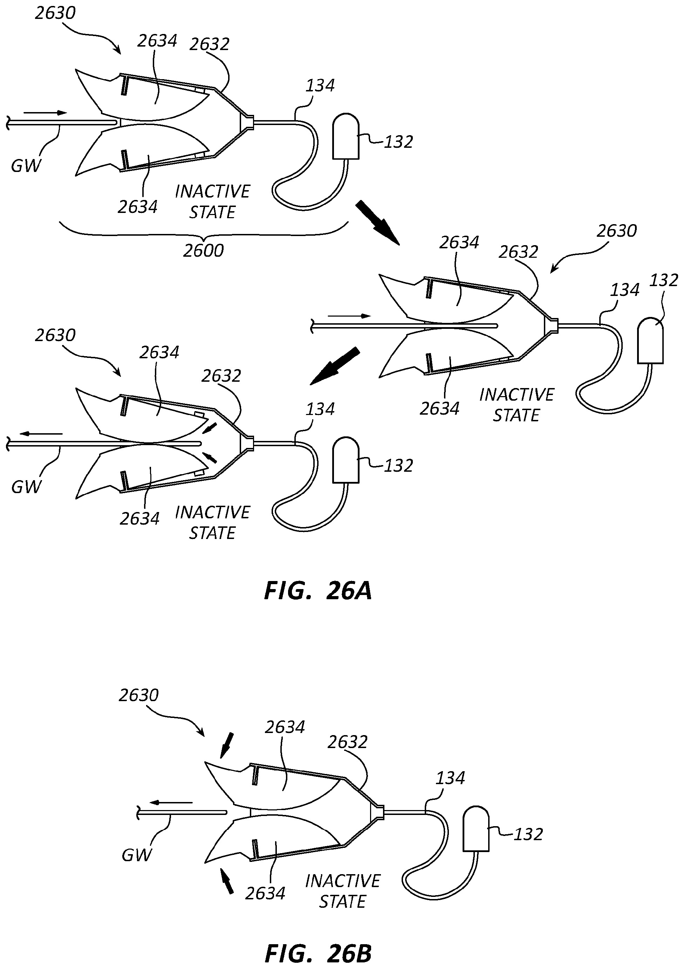

FIG. 26A illustrates a twenty-fourth connection system 2600 including a twenty-fourth probe connector 2630 in accordance with some embodiments. FIG. 26B illustrates disengaging a wire probe from the twenty-fourth probe connector 2630 in accordance with some embodiments. As shown, the probe connector 2630 is a self-actuatable probe connector 2630 including a housing 2632 having an opening in a distal end of the housing 2632 and a pair of cams 2634 mounted on camshafts within the housing 2632, wherein the pair of cams 2634 also has an opening between the pair of cams 2634 commensurate with or extending past the distal end of the housing 2632. A radius of a portion of a cam within the housing 2632 is greater than a radius of a portion of the cam extending past the distal end of the housing 2632. In addition, the pair of cams 2634 serves as a pair of electrical contacts because each cam of the pair of cams 2634 is either formed of one or more metals or lined with the one or more metals.

When the probe connector 2630 is in an inactive state (e.g., the pair of cams 2634 is available to accept a wire probe), the wire probe can be inserted into the distal end of the housing 2632 between the pair of cams 2634. When the probe connector 2630 is in an active state, the wire probe is tightly lodged within the housing 2632 between the pair of cams 2634. The wire probe cannot be directly withdrawn from the housing 2632 due to a self-actuatable mechanism actuated by pulling the wire probe from the distal end of the housing 2632. The self-actuatable mechanism is due to the radius of the portion of each cam within the housing 2632 being larger than the radius of the portion of each cam extending past the distal end of the housing 2632, which produces a clamping force *(see* arrows in FIG. 26A) on the wire probe when the wire probe is directly withdrawn from the housing 2632. Thus, in the active state, the wire probe is lodged within the housing 2632 between the pair of cams 2634 with a sufficient frictional force to secure the wire probe in the probe connector 2630 and provide an electrical connection to the tether 134. However, the wire probe can be indirectly withdrawn from between the pair of cams 2634 by pushing the portion of each cam extending past the distal end of the housing 2632 toward the other cam. Pushing on the pair of cams 2634 as such rotates each cam on its camshaft, thereby separating the pair of cams 2634 and allowing the wire probe to be withdrawn.

The probe connector 2630 can be formed of one or more metals including alloys, one or more polymers including co-polymers or polymer blends, or a combination thereof. For example, the housing 2632 and the pair of cams 2634 can be formed of the one or more polymers while the one or more metals can line the pair of cams 2634. The metal-lined pair of cams 2634 can be coupled to the one or more wires of the tether 134 such that the wire probe forms an electrical connection to the one or more wires of the tether 134 when the wire probe is lodged between the pair of cams 2634.

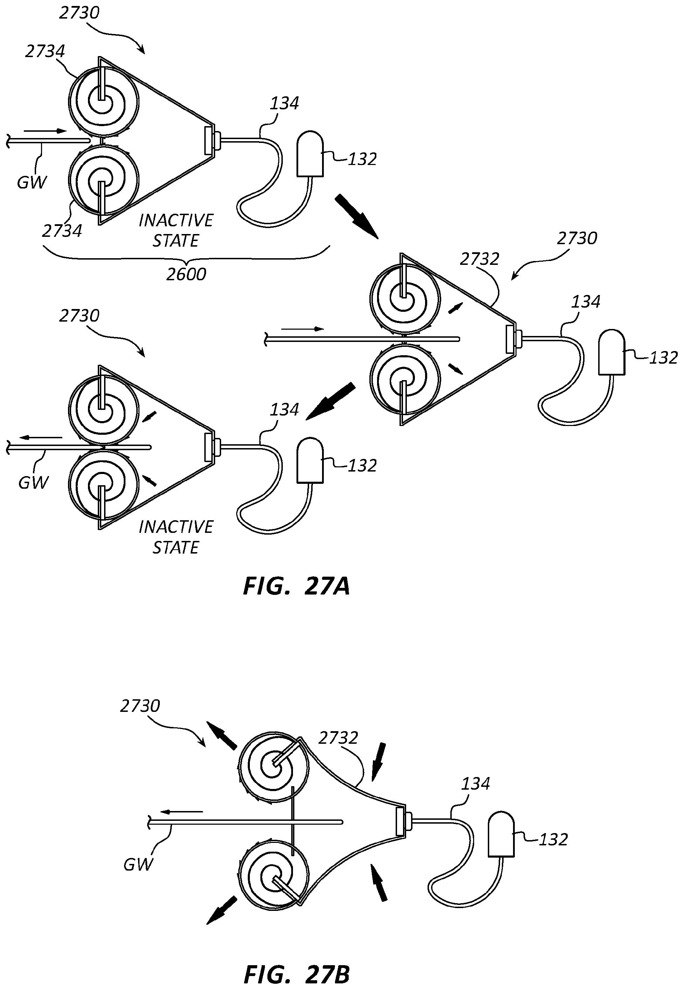

FIG. 27A illustrates a twenty-fifth connection system 2700 including a twenty-fifth probe connector 2730 in accordance with some embodiments. FIG. 27B illustrates disengaging a wire probe from the twenty-fifth probe connector 2730 in accordance with some embodiments. As shown, the probe connector 2730 is a self-actuatable probe connector 2730 including a housing 2732 having an opening in a distal end of the housing 2732 and a pair of wheels 2734 mounted on shafts within the housing 2732, wherein the pair of wheels 2734 also has an opening between the pair of wheels 2734 commensurate with or extending past the distal end of the housing 2732. A tension spring or extension spring (not shown), or a pair of such springs, mounted within the housing 2732 (e.g., between the shafts of the pair of wheels 2734) pulls the pair of wheels 2734 toward each other. In addition, the pair of wheels 2734 serves as a pair of electrical contacts because each wheel of the pair of wheels 2734 includes teeth of one or more metals.

When the probe connector 2730 is in an inactive state, the wire probe can be inserted into the distal end of the housing 2732 between the pair of wheels 2734. While the pair of wheels 2734 are pulled toward each other by the tension spring or extension spring, or the pair of such springs, the wheels can roll on the shafts to allow insertion of the wire probe between the pair of wheels 2734. When the probe connector 2730 is in an active state, the wire probe is tightly lodged within the housing 2732 between the pair of wheels 2734. The wire probe cannot be directly withdrawn from the housing 2732 due to a self-actuatable mechanism actuated by pulling the wire probe from the distal end of the housing 2732. The self-actuatable mechanism is due to the shafts upon which the pair of wheels 2734 are mounted turning in only one direction (e.g. counterclockwise and clockwise respectively for the top wheel and the bottom wheel of the pair of wheels 2734 shown in FIG. 27A). In some embodiments, the shafts are configured to turn in only one direction by way of a ratchet mechanism disposed within the housing 2732 that utilizes the teeth on the pair of wheels 2734 as pawls. Thus, in the active state, the wire probe is lodged within the housing 2732 between the pair of wheels 2734 with a sufficient frictional force to secure the wire probe in the probe connector 2730 and provide an electrical connection by way of the teeth to the tether 134. However, the wire probe can be indirectly withdrawn from between the pair of wheels 2734 by compressing the housing 2732 adjacent the pair of wheels 2734. *(See* arrows of FIG. 27B.) Compressing the housing 2732 as such deforms the tension spring or extension spring, or the pair of such springs, by extension thereof, thereby separating the pair of wheels 2734 and allowing the wire probe to be withdrawn.

The probe connector 2730 can be formed of one or more metals including alloys, one or more polymers including co-polymers or polymer blends, or a combination thereof. For example, the housing 2732 and the pair of wheels 2734 can be formed of the one or more polymers while the teeth of the pair of wheels 2734 can be formed of the one or more metals. The teeth can be coupled to the one or more wires of the tether 134 such that the wire probe forms an electrical connection to the one or more wires of the tether 134 when the wire probe is lodged between the pair of wheels 2734.

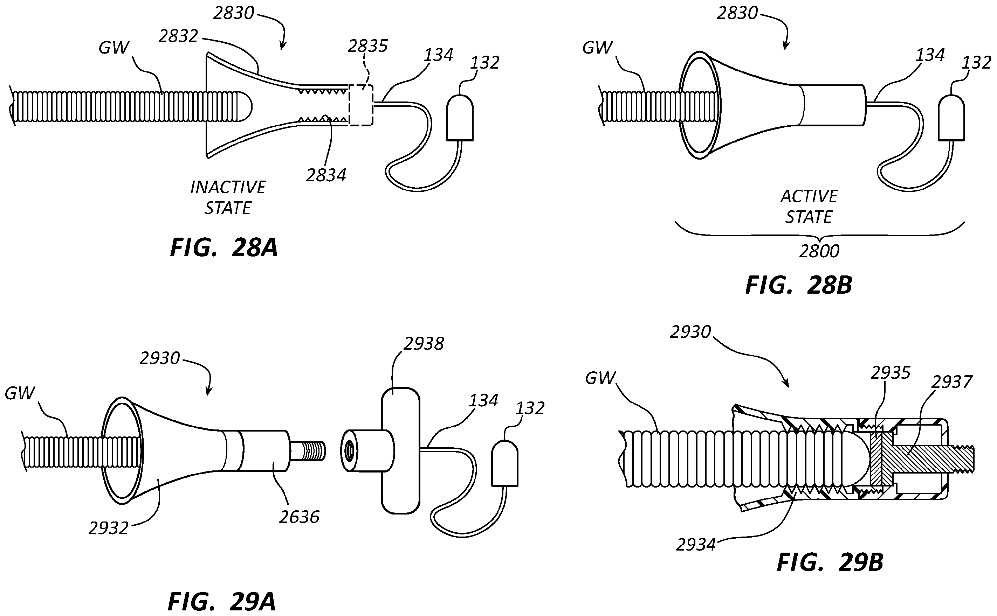

FIG. 28A illustrates a twenty-sixth connection system 2800 including a twenty-sixth probe connector 2830 in accordance with some embodiments. FIG. 28B illustrates a longitudinal cross section of the twenty-sixth probe connector 2830 in accordance with some embodiments. As shown, the probe connector 2830 includes a housing 2832 having a probe-guiding funnel-shaped opening in a distal end of the housing 2832 and a set of threads 2834 within the housing 2832 in a proximal end portion of the housing 2832 forming a threadable substrate configured for screwing a wire probe into the housing 2832. The set of threads 2834 can be formed of one or more metals such that the set of threads 2834 provides both a mechanical connection and an electrical connection for the wire probe. Alternatively, the set of threads 2834 can be formed of one or more polymers such that the set of threads 2834 provides only a mechanical connection for the wire probe. With respect to an electrical connection for the wire probe in such embodiments, the housing 2832 further incudes an electrical contact 2835 in a proximal end of the housing proximal to the set of threads 2834 to provide the electrical connection.

FIG. 29A illustrates a twenty-seventh connection system 2900 including a twenty-seventh probe connector 2930 in accordance with some embodiments. FIG. 29B illustrates a longitudinal cross section of the twenty-seventh probe connector 2930 in accordance with some embodiments. Like the probe connector 2830, the probe connector 2930 includes a housing 2932 having a probe-guiding funnel-shaped opening in a distal end of the housing 2932 and a set of threads 2934 within the housing 2932 in a proximal end portion of the housing 2932 forming a threadable substrate configured for screwing a wire probe into the housing 2932. And like the probe connector 2830, the set of threads 2934 of the probe connector 2930 can be formed of either one or more metals or one or more polymers depending upon whether both a mechanical connection and an electrical connection is desired for the wire probe or only the mechanical connection. Different than the probe connector 2830, the probe connector 2930 includes an adapter 2936 having a plug configured to engage a socket or receptacle component 2938 coupled to the tether 134 for customizing the probe connector 2930 to various systems having such socket or receptacle components. In some embodiments in which the set of threads 2934 is formed of the one or more polymers, the housing 2932 further incudes an electrical contact 2935 in a proximal end of the housing 2932 proximal to the set of threads 2934 as shown in FIG. 29B to provide the electrical connection. In embodiments such as the foregoing, or in the embodiments in which the set of threads 2934 are formed of the one or more metals, the adapter 2936 includes an electrical contact 2937 configured to couple to the electrical contact 2935 or the set of threads 2934 when made of the one or more metals. Thereby, the adapter 2936 is configured to mechanically and electrically connect the probe connector 2930 to various systems.

With respect to each probe connector of the probe connectors 2830 and 2930, when the probe connector 2830, 2930 is in an inactive state without a wire probe, the wire probe can be inserted into the distal end of the housing 2832, 2932 and screwed into the set of threads 2834, 2934. When the probe connector 2830, 2930 is in an active state, the wire probe is interlocked with the set of threads 2834, 2934 within the housing 2832, 2932 and cannot be withdrawn without unscrewing the wire probe from the set of threads 2834, 2934. Thus, in the active state, the wire probe is interlocked with the set of threads 2834, 2934 and sufficiently secured within the housing 2832, 2932 with at least a mechanical connection. An electrical connection in the active state of the probe connector 2830, 2930 is provided by way of either the set of threads 2834, 2934 or the guide wire abutting the electrical contact 2835, 2935 when screwed into the set of threads 2834, 2934.

The probe connector 2830, 2930 can be formed of one or more metals including alloys, one or more polymers including co-polymers or polymer blends, or a combination thereof. For example, the housing 2832, 2932 and the set of threads 2834, 2934 can be formed of the one or more polymers while the electrical contact 2835, 2935 can be formed of the one or more metals. Alternatively, the set of threads 2834, 2934 can be formed of the one or more metals obviating the electrical contact 2835, 2935. The set of threads 2834, 2934 or the electrical contact 2835, 2935 can be directly coupled to the one or more wires of the tether 134 or indirectly coupled to the one or more wires of the tether 134 through the adapter 2936 and the socket or receptacle component 2938 such that the wire probe forms an electrical connection to the one or more wires of the tether 134 when the wire probe is screwed into the set of threads 2834, 2934.

FIG. 30A illustrates a twenty-eighth connection system 3000 including a twenty-eighth probe connector 3030 in an inactive state in accordance with some embodiments. FIG. 30B illustrates the twenty-eighth connection system 3000 including the twenty-eighth probe connector 3030 in an active state in accordance with some embodiments. As shown, the probe connector 3030 is integrated with the catheter 72. *(See* also FIGS. 2 and 3B.) The probe connector 3030 includes an adapter 3032 configured to couple to a luer connector 74 of the catheter 72 and a clamp 3034 configured to clamp a wire probe in the clamp 3034. A housing 3036 of the adapter 3032 includes a slot through which a plate 3038 having a cutout 3039 configured for the wire probe can slide in or out of the housing 3036 adj acent the luer connector 74. The clamp 3034 is configured with a tension or extension spring to clamp around a housing 3036 of the adapter 3032.

When the probe connector 3030 is in an inactive state with the wire probe extending from the luer connector, the adapter 3032 coupled to the luer connector 74, and jaws of the clamp 3034 clamped around the housing 3036 of the adapter 3032, the wire probe can be inserted into a back of a mouth of the clamp 3034 to dislodge the clamp 3034 from the adapter 3032 and clamp the wire probe in the clamp 3034. If the clamp 3034 is coupled to the plate 3038 such as by a breakable tether to the plate 3038 or a jaw of the clamp 3034 extending through the cutout 3039 of the plate, the plate 3038 is configured to slide into the housing 3036 of the adapter 3032 and capture the wire probe distal to the clamp 3034 upon the wire probe dislodging the clamp 3034 from the adapter 3032. Otherwise, the plate 3038 can be pushed in the housing 3036 of the adapter 3032 after the clamp 3034 clamps the wire probe. When the probe connector 3030 is in an active state the jaws of the clamp 3034 are clamped around the wire probe to provide both a mechanical connection and an electrical connection. In addition, the wire probe is inserted in the plate 3038, which provides a secondary mechanical connection. While in the active state, the wire probe is clamped with a sufficient clamping force to secure the wire probe in the probe connector 3030 and provide an electrical connection to the tether 134. In addition, the secondary mechanical connection provided by the plate 3038 ensures wire-probe embolisms are prevented.

Any probe connector, including the probe connectors described herein, can be configured with an adapter such as the adapter 3032 to couple to the luer connector 74 of the catheter 72. A wire probe can be inserted into such a probe connector through its adapter to dislodge the probe connector from the adapter, thereby mechanically and electrically connecting the wire probe to the probe connector.

The probe connector 3030 can be formed of one or more metals including alloys, one or more polymers including co-polymers or polymer blends, or a combination thereof. For example, the housing 3036 and the plate 3038 of the adapter 3032 can be formed of the one or more polymers. The jaws of the clamp 3034 can also be formed of the one or more polymers provided one or more electrical contacts disposed in the jaws of the clamp 3034 are formed of the one or more metals. Otherwise, the jaws of the clamp 3034 can be formed of the one or more metals. The jaws of the clamp 3034, or the one or more electrical contacts thereof, can be coupled to the one or more wires of the tether 134 such that the wire probe forms an electrical connection to the one or more wires of the tether 134 when the wire probe is clamped within the clamp 3034.

Advantages of the foregoing probe connectors include providing viable electrical connections, effective mechanical connections, time savings, and improved safety.

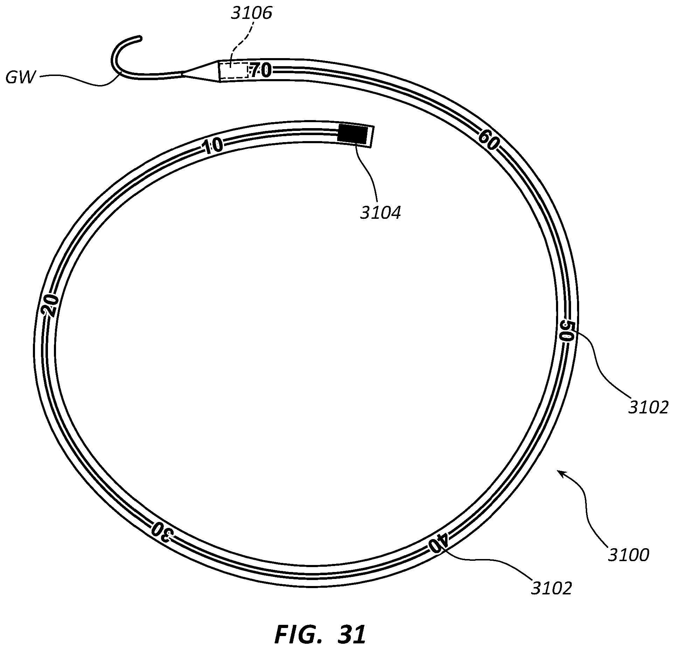

FIG. 31 illustrates a wire-probe dispenser 3100 in accordance with some embodiments. Sometimes it can be a challenge for users to know how deeply a wire probe is inserted in a patient when markings on the wire probe corresponding to depths are covered with fluid (e.g., blood), hidden within a wire-probe dispenser such that the markings cannot be viewed, or both. As shown, the wire-probe dispenser 3100 includes easily viewed markings 3102 on an abluminal surface of the wire-probe dispenser 3100 to aid such users in determining how deeply a wire probe is inserted in a patient. The markings 3102 on the wire-probe dispenser 3100 include graduations combined with numbers that increase from a proximal end of the wire-probe dispenser 3100 to a distal end of the wire-probe dispenser 3100. In addition to the markings 3102, a proximal end of the wire probe disposed in a lumen of the wire-probe dispenser 3100 is fitted with a marker 3104 such as a small molded piece around the proximal end of the wire probe, which marker 3104 is visible through a body of the wire-probe dispenser 3100. As a distal end of the wire probe is pulled from the wire-probe dispenser 3100, the marker 3104 at the proximal end of the wire probe is simultaneously pulled along the lumen of the wire-probe dispenser 3100 beneath the markings 3102, thereby marking how deeply the wire probe is inserted in a patient as the wire probe is inserted in the patient. The distal end of the wire-probe dispenser 3100 can be tapered to form a constriction that disengages the marker 3104 from the wire probe when the wire probe is completely pulled out from the wire-probe dispenser 3100, or the wire-probe dispenser 3100 can include a stop 3106 disposed in the distal end of the wire-probe dispenser 3100 to the same effect as the constriction.

While some particular embodiments have been disclosed herein, and while the particular embodiments have been disclosed in some detail, it is not the intention for the particular embodiments to limit the scope of the concepts provided herein. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the concepts provided herein.

## Claims

1. A connection system for establishing electrical connections across a sterile field, the connection system comprising:
a sensor connector (132) including a piercing element, the piercing element configured to pierce a sterile side of a drape and establish a first electrical connection with a receptacle of a sensor when the piercing element is inserted into the receptacle;
a user-actuatable probe connector (1930-) including:
a housing (1932) configured for insertion of a wire probe (GW) therein, the wire probe being a guidewire; and
a probe-connecting means configured for a user to establish a second electrical connection between one or more electrical contacts of the probe connector (1930) and the wire probe (GW), the probe-connecting means is an actuatable device including an actuator (1938) configured to actuate a connecting mechanism of the actuatable device for establishing the second electrical connection between the electrical contact(s) of the probe connector (1930) and the wire probe (GW) within the housing (1932); and
a tether (134) having a first end connected to the sensor connector (132) and a second end connected to the probe connector (1930), the tether (134) configured to convey electrical signals between the sensor connector (132) and the probe connector (1930).

2. The connection system of claim 1, wherein the probe connecting means is formed of a metal or a polymer, the probe-connecting means formed of the polymer having the electrical contact disposed therein for establishing the second electrical connection with the wire probe (GW).

3. The connection system of claim 1 or claim 2, wherein the actuator is a knob or handle (1938) extending from a side of the housing (1932), the knob or handle configured to rotate until the second electrical connection is established between the electrical contact(s) within the housing (1932) and the wire probe (GW).

4. The connection system of claim 1 or claim 2, wherein the actuator is a slide button (2334) extending from a side of the housing (2332), the slide button configured to establish the second electrical connection between the electrical contact within the housing (2332) and the wire probe (GW) when the slide button is slid from a first side of a channel (2335) in the side of the housing (2332) to a second side of the channel (2335) opposite the first side.

5. The connection system of claim 1 or claim 2, wherein the actuator (2434) is a twistable end portion of the housing (2432), the twistable end portion configured to twist until the second electrical connection is established between a diaphragmatic arrangement of blade-like electrical contacts (2436) as the electrical contact(s) within the housing and the wire probe (GW), which wire probe (GW) is tightly lodged within the diaphragmatic arrangement of blade-like electrical contacts (2436) in an active state.

6. The connection system of either claim 1 or 6, wherein the actuator is a push button (2134) extending from a side of the housing (2132), the push button (2134) configured to lodge the wire probe (GW) against the electrical connector(s) on an inner surface of the housing (2132) with sufficient frictional force to establish a mechanical connection as well as the second electrical connection between the electrical contact(s) within the housing (2132) and the wire probe (GW) when the push button is pushed.

7. The connection system of claim 3, wherein the electrical contact(s) (1934, 1936) transition from sandwiching the wire probe (GW) between the electrical contact(s) (1934, 1936) within the housing (1932) in an inactive state before the actuator (1938) actuates the connecting mechanism to clamping the wire probe (GW) between the electrical contact(s) (1934 1936) with a sufficient clamping force to secure the wire probe (GW) in the probe connector (1930) in an active state after the actuator (1938) actuates the connecting mechanism, thereby providing the second electrical connection.

8. The connection system of claim 3, wherein the electrical contact(s) (2036) are two prongs within the housing (2032) that rotate through a same angle as the actuator (2034) when the connecting mechanism is activated by rotating the actuator (2034), the wire probe (GW) between the electrical contact(s) (2036) within the housing (2032) in an inactive state before the actuator (2034) is rotated, and the wire probe (GW) tortuously disposed or slightly twisted between the electrical contact(s) (2036) within the housing (2032) in an active state after the actuator (2034) is rotated.

9. The connection system of claim 4, wherein a luminal surface of the housing (2332) includes spiral grooves (2333) that spiral from a proximal end to a distal end of the housing (2332) for directing the electrical contact(s) (2336) as conductive filaments or wires along the luminal surface of the housing (2332) when moved therethrough in accordance with the slide button (2334), the wire probe (GW) fitting loosely within the electrical contact(s) (2336) in an inactive state, and the wire probe (GW) tightly lodged within the electrical contacts (2336) in an active state, thereby providing the second electrical connection.

## Patentansprüche

1. Verbindungssystem zur Herstellung elektrischer Verbindungen über ein steriles Feld hinweg, das Verbindungssystem umfassend:
einen Sensorverbinder (132), einschließend ein Durchstichelement, wobei das Durchstichelement dazu konfiguriert ist, eine sterile Seite eines sterilen Abdecktuchs zu durchstechen und eine erste elektrische Verbindung mit einer Aufnahme eines Sensors herzustellen, wenn das Durchstichelement in die Aufnahme eingeführt wird;
einen benutzerbetätigbaren Sondenverbinder (1930), einschließend:
ein Gehäuse (1932), das zum Einführen einer Drahtsonde (GW) darin konfiguriert ist, wobei die Drahtsonde ein Führungsdraht ist; und
Sondenanschlussmittel, das dazu konfiguriert sind, dass ein Benutzer eine zweite elektrische Verbindung zwischen einem oder mehreren elektrischen Kontakten des Sondenverbinders (1930) und der Drahtsonde (GW) herstellt, wobei das Sondenanschlussmittel eine betätigbare Vorrichtung ist, die einen Aktuator (1938) einschließt, der dazu konfiguriert ist, einen Verbindungsmechanismus der betätigbaren Vorrichtung zu betätigen, um die zweite elektrische Verbindung zwischen einem oder mehreren elektrischen Kontakten des Sondenverbinders (1930) und der Drahtsonde (GW) innerhalb des Gehäuses (1932) herzustellen; und
eine Verbindungsleitung (134) aufweisend ein erstes Ende, das mit dem Sensorverbinder (132) verbunden ist, und ein zweites Ende, das mit dem Sondenverbinder (1930) verbunden ist, wobei die Verbindungsleitung (134) dazu konfiguriert ist, elektrische Signale zwischen dem Sensorverbinder (132) und dem Sondenverbinder (1930) zu übertragen.

2. Verbindungssystem nach Anspruch 1, wobei das Sondenanschlussmittel aus einem Metall oder einem Polymer gebildet ist, wobei das aus dem Polymer gebildete Mittel den elektrischen Kontakt darin angeordnet aufweisen, um die zweite elektrische Verbindung mit der Drahtsonde (GW) herzustellen.

3. Verbindungssystem nach Anspruch 1 oder Anspruch 2, wobei der Aktuator ein Knopf oder Griff (1938) ist, der sich von einer Seite des Gehäuses (1932) erstreckt und dazu konfiguriert ist, sich zu drehen, bis die zweite elektrische Verbindung zwischen den innerhalb des Gehäuses (1932) angeordneten elektrischen Kontakten und der Drahtsonde (GW) hergestellt ist.

4. Verbindungssystem nach Anspruch 1 oder Anspruch 2, wobei der Aktuator ein Schieber (2334) ist, der sich von einer Seite des Gehäuses (2332) erstreckt, wobei der Schieber dazu konfiguriert ist, die zweite elektrische Verbindung zwischen dem elektrischen Kontakt innerhalb des Gehäuses (2332) und der Drahtsonde (GW) herzustellen, wenn der Schieber von einer ersten Seite eines Gleitkanals (2335) in einer Seitenwand des Gehäuses (2332) auf die der ersten Seite gegenüberliegende zweite Seite des Kanals (2335) verschoben wird.

5. Verbindungssystem nach Anspruch 1 oder Anspruch 2, wobei der Aktuator (2434) ein verdrehbarer Endabschnitt des Gehäuses (2432) ist, wobei der verdrehbare Endabschnitt dazu konfiguriert ist, sich zu verdrehen, bis die zweite elektrische Verbindung zwischen einer membranartigen Anordnung klingenförmiger elektrischer Kontakte (2436), die als elektrische Kontakte innerhalb des Gehäuses dienen, und der Drahtsonde (GW) hergestellt ist, wobei die Drahtsonde (GW) in einem aktiven Zustand innerhalb der membranartigen Anordnung klingenförmiger elektrischer Kontakte (2436) festgeklemmt ist.

6. Verbindungssystem nach Anspruch 1 oder 6, wobei der Aktuator ein Druckknopf (2134) ist, der sich von einer Seite des Gehäuses (2132) erstreckt, wobei der Druckknopf (2134) dazu konfiguriert ist, die Drahtsonde (GW) mit ausreichender Reibkraft gegen mindestens einen elektrischen Verbinder an einer Innenfläche des Gehäuses (2132) festzuklemmen, um eine mechanische Verbindung sowie die zweite elektrische Verbindung zwischen den elektrischen Kontakten innerhalb des Gehäuses (2132) und der Drahtsonde (GW) herzustellen, wenn der Druckknopf gedrückt wird.

7. Verbindungssystem nach Anspruch 3, wobei die elektrischen Kontakte (1934, 1936) von einem inaktiven Zustand, in dem die Drahtsonde (GW) innerhalb des Gehäuses (1932) zwischen den elektrischen Kontakten (1934, 1936) eingespannt ist, bevor der Aktuator (1938) den Verbindungsmechanismus betätigt, in einen aktiven Zustand übergehen, in dem die Drahtsonde (GW) nach Betätigung des Verbindungsmechanismus durch den Aktuator (1938) zwischen den elektrischen Kontakten (1934, 1936) mit ausreichender Klemmkraft festgeklemmt ist, um die Drahtsonde (GW) im Sondenverbinder (1930) zu sichern, wodurch die zweite elektrische Verbindung bereitgestellt wird.

8. Verbindungssystem nach Anspruch 3, wobei der/die elektrischen Kontakt(e) (2036) zwei Kontaktzungen innerhalb des Gehäuses (2032) sind, die sich um den gleichen Winkel drehen wie der Aktuator (2034), wenn der Verbindungsmechanismus durch Drehen des Aktuators (2034) aktiviert wird, wobei sich die Drahtsonde (GW) in einem inaktiven Zustand vor dem Drehen des Aktuators (2034) zwischen den elektrischen Kontakten (2036) innerhalb des Gehäuses (2032) befindet und die Drahtsonde (GW) in einem aktiven Zustand nach dem Drehen des Aktuators (2034) mäanderförmig oder leicht verdreht zwischen den elektrischen Kontakten (2036) innerhalb des Gehäuses (2032) angeordnet ist.

9. Verbindungssystem nach Anspruch 4, wobei eine luminale Oberfläche des Gehäuses (2332) spiralförmige Rillen (2333) einschließt, die sich von einem proximalen Ende zu einem distalen Ende des Gehäuses (2332) spiralförmig erstrecken, um die elektrischen Kontakte (2336) als leitfähige Filamente oder Drähte entlang der luminalen Oberfläche des Gehäuses (2332) zu führen, wenn diese in Übereinstimmung mit der Betätigung des Schiebers (2334) durch die Rillen geführt werden, wobei die Drahtsonde (GW) in einem inaktiven Zustand lose in den elektrischen Kontakten (2336) sitzt und die Drahtsonde (GW) in einem aktiven Zustand in den elektrischen Kontakten (2336) festgeklemmt ist, wodurch die zweite elektrische Verbindung bereitgestellt wird.

## Revendications

1. Système de connexion pour établir des connexions électriques à travers un champ stérile, le système de connexion comprenant :
un connecteur de capteur (132) incluant un élément de perçage, l'élément de perçage étant configuré pour percer un côté stérile d'un drap et établir une première connexion électrique avec un réceptacle d'un capteur lorsque l'élément de perçage est inséré dans le réceptacle ;
un connecteur de sonde actionnable par l'utilisateur (1930-) incluant :
un boîtier (1932) configuré pour une insertion d'une sonde filaire (GW) en son sein, la sonde filaire étant un fil-guide ; et
un moyen de raccordement de sonde configuré pour permettre à un utilisateur d'établir une seconde connexion électrique entre un ou plusieurs contacts électriques du connecteur de sonde (1930) et la sonde filaire (GW), le moyen de raccordement de sonde est un dispositif actionnable incluant un actionneur (1938) configuré pour actionner un mécanisme de raccordement du dispositif actionnable afin d'établir la seconde connexion électrique entre le ou les contact(s) électrique(s) du connecteur de sonde (1930) et la sonde filaire (GW) à l'intérieur du boîtier (1932) ; et
un cordon (134) présentant une première extrémité reliée au connecteur de capteur (132) et une seconde extrémité reliée au connecteur de sonde (1930), le cordon (134) étant configuré pour véhiculer des signaux électriques entre le connecteur de capteur (132) et le connecteur de sonde (1930).

2. Système de connexion selon la revendication 1, dans lequel le moyen de raccordement de sonde est formé d'un métal ou d'un polymère, le moyen de raccordement de sonde formé du polymère présentant le contact électrique disposé en son sein pour établir la seconde connexion électrique avec la sonde filaire (GW).

3. Système de connexion selon la revendication 1 ou la revendication 2, dans lequel l'actionneur est un bouton ou une poignée (1938) s'étendant depuis un côté du boîtier (1932), le bouton ou la poignée étant configuré(e) pour tourner jusqu'à ce que la seconde connexion électrique soit établie entre le ou les contact(s) électrique(s) à l'intérieur du boîtier (1932) et de la sonde filaire (GW).

4. Système de connexion selon la revendication 1 ou la revendication 2, dans lequel l'actionneur est un bouton coulissant (2334) s'étendant depuis un côté du boîtier (2332), le bouton coulissant étant configuré pour établir la seconde connexion électrique entre le contact électrique à l'intérieur du boîtier (2332) et la sonde filaire (GW) lorsque le bouton coulissant est amené à coulisser depuis un premier côté d'un canal (2335) dans le côté du boîtier (2332) jusqu'à un second côté du canal (2335) opposé au premier côté.

5. Système de connexion selon la revendication 1 ou la revendication 2, dans lequel l'actionneur (2434) est une partie d'extrémité torsadable du boîtier (2432), la partie d'extrémité torsadable étant configurée pour se tordre jusqu'à ce que la seconde connexion électrique soit établie entre un agencement à diaphragme de contacts électriques à lame (2436), qui constituent le ou les contact(s) électrique(s) à l'intérieur du boîtier, et la sonde filaire (GW), laquelle sonde filaire (GW) est fermement logée dans l'agencement à diaphragme de contacts électriques à lame (2436) dans un état actif.

6. Système de connexion selon la revendication 1 ou la revendication 6, dans lequel l'actionneur est un bouton-poussoir (2134) s'étendant depuis un côté du boîtier (2132), le bouton-poussoir (2134) étant configuré pour loger la sonde filaire (GW) contre le ou les connecteur(s) électrique(s) sur une surface interne du boîtier (2132) avec une force de friction suffisante pour établir une liaison mécanique ainsi que la seconde connexion électrique entre le ou les contact(s) électrique(s) à l'intérieur du boitier (2132) et la sonde filaire (GW) lorsque le bouton-poussoir est enfoncé.

7. Système de connexion selon la revendication 3, dans lequel le ou les contact(s) électrique(s) (1934, 1936) passent d'un état inactif dans lequel la sonde filaire (GW) est prise en sandwich entre le ou les contact(s) électrique(s) (1934, 1936) à l'intérieur du boîtier (1932) avant que l'actionneur (1938) n'actionne le mécanisme de raccordement, à un état actif dans lequel la sonde filaire (GW) est serrée entre le ou les contact(s) électrique(s) (1934, 1936) avec une force de serrage suffisante pour immobiliser la sonde filaire (GW) dans le connecteur de sonde (1930) après que l'actionneur (1938) a actionné le mécanisme de raccordement, fournissant ainsi la seconde connexion électrique.

8. Système de connexion selon la revendication 3, dans lequel le ou les contact(s) électrique(s) (2036) sont deux broches à l'intérieur du boîtier (2032) qui tournent d'un même angle que l'actionneur (2034) lorsque le mécanisme de raccordement est activé en faisant tourner l'actionneur (2034), la sonde filaire (GW) étant entre le ou les contact(s) électrique(s) (2036) à l'intérieur du boîtier (2032) dans un état inactif avant que l'actionneur (2034) ne soit tourné, et la sonde filaire (GW) étant disposée de manière tortueuse ou légèrement torsadée entre le ou les contact(s) électrique(s) (2036) à l'intérieur du boîtier (2032) dans un état actif après que l'actionneur (2034) a été tourné.

9. Système de connexion selon la revendication 4, dans lequel une surface luminale du boîtier (2332) inclut des rainures en spirale (2333) qui s'étendent en spirale d'une extrémité proximale à une extrémité distale du boîtier (2332) pour diriger le ou les contact(s) électrique(s) (2336), sous forme de filaments ou fils conducteurs, le long de la surface luminale du boîtier (2332) lorsqu'ils s'y déplacent en fonction du bouton coulissant (2334), la sonde filaire (GW) s'ajustant librement dans le ou les contact(s) électrique(s) (2336) dans un état inactif, et la sonde filaire (GW) étant fermement logée dans le ou les contact(s) électrique(s) (2336) dans un état actif, fournissant ainsi la seconde connexion électrique.
